# EUROPEAN PATENT SPECIFICATION

(11) **EP 3 919 017 B1**
(45) Date of publication and mention of the grant of the patent: **27.08.2025**
(21) Application number: 21177611.7
(22) Date of filing: 03.06.2021
(51) Int. Cl.: A61B 34/20, A61B 34/30

(54) **SURGICAL SYSTEM COMPRISING A MACHINE LEARNING SYSTEM FOR NAVIGATED SPINAL SURGERIES**
CHIRURGISCHES SYSTEM MIT MASCHINELL LERNENDEM SYSTEM FÜR NAVIGIERTE WIRBELSÄULENOPERATIONEN
SYSTÈME CHIRURGICAL À APPRENTISSAGE AUTOMATIQUE POUR LES CHIRURGIES RACHIDIENNES NAVIGUÉES

(30) Priority: 03.06.2020 US 202016891870
(43) Date of publication of application: 08.12.2021
(73) Proprietor: Globus Medical, Inc., Audubon, PA 19403 (US)
(72) Inventor: PAUL, David C., Phoenixville, 19460 (US); JOHNSON, Norbert, North Andover, 01845 (US); GLERUM, Chad, Pennsburg, 18073 (US)
(74) Representative: Morabito, Sara

(56) References cited:
- WO-A1-2020/079598
- WO-A1-2021/068933
- US-A1- 2019 254 750
- US-A1- 2019 262 084
- US-A1- 2020 038 109
- US-A1- 2020 078 180
- CHANG MICHAEL ET AL: "The Role of Machine Learning in Spine Surgery: The Future Is Now", FRONTIERS IN SURGERY, vol. 7, 1 January 2020 (2020-01-01), pages 54, XP055852278, Retrieved from the Internet <URL:https://www.ncbi.nlm.nih.gov/pmc/articles/PMC7472375/pdf/fsurg-07-00054.pdf> DOI: 10.3389/fsurg.2020.00054
- LAFAGE RENAUD ET AL: "Self-learning computers for surgical planning and prediction of postoperative alignment", EUROPEAN SPINE JOURNAL, SPRINGER VERLAG, BERLIN, DE, vol. 27, no. 1, 9 February 2018 (2018-02-09), pages 123 - 128, XP036434876, ISSN: 0940-6719, [retrieved on 20180209], DOI: 10.1007/S00586-018-5497-0
- ANONYMOUS: "Surgical planning - Wikipedia", 22 March 2021 (2021-03-22), pages 1 - 3, XP093084195, Retrieved from the Internet <URL:https://en.wikipedia.org/w/index.php?title=Surgical_planning&oldid=1013562732> [retrieved on 20230921]

## Description

### FIELD

The present disclosure relates to medical devices and systems, and more particularly, providing navigation information to users and/or surgical robots for spine surgeries.

### BACKGROUND

The vertebrate spine is the axis of the skeleton providing structural support for other parts of the body. Adjacent vertebrae of the spine are supported by an intervertebral disc, which serves as a mechanical cushion permitting controlled motion between vertebral segments of the axial skeleton. The intervertebral disc is a unique structure comprised of three components: the nucleus pulposus ("nucleus"), the annulus fibrosus ("annulus") and two vertebral end plates.

The spinal disc can be displaced or damaged due to trauma, disease, degenerative defects or wear over an extended period of time. For example, disc herniation occurs when annulus fibers are weakened or torn and the inner tissue of the nucleus becomes permanently bulged. The mass of a herniated or "slipped" nucleus tissue can compress a spinal nerve, resulting in leg pain, loss of muscle control, or even paralysis. In addition, in some cases, a degenerated nucleus can lose its water binding ability and deflate, thereby reducing the height of the nucleus and causing the annulus to buckle in certain areas.

To alleviate back pain caused by disc herniation or degeneration, the disc can be removed and replaced by an implant that promotes fusion of the remaining bone anatomy. The implant, such as a spacer or cage body, should be sufficiently strong to support the spine under a wide range of loading conditions. The implant should also be configured so that it is likely to remain in place once it has been positioned in the spine by the surgeon. In addition, the implant should be capable of being delivered minimally invasively or at least through a relatively small incision into a desired position.

The degree of surgical outcome success is dependent on the surgeon's ability to design and implement the best surgical plan for a particular patient's needs. However, optimal design and implementation may not be achievable in view of inherent limitations in a surgeon's ability to adapt past practice experiences to a patient's particular spinal geometries and corrective needs when selecting among a myriad of combinations of medically accepted spinal surgery procedures and numerous available implants types and configurations.

Thus, there remains a need for an improved process that addresses these difficulties.

US 2020/078180 A1 discloses a computer-implemented method for manufacturing an orthopedic implant, employing a surgical assistance system to apply analysis procedures by supplying implant surgery information to a machine learning model trained to select implant configurations and to provide output indicating a pedicle screw configuration. The disclosed system further includes a display in the form of a holographic, or augmented reality display, such as a heads-up display device or a head-mounted device. The disclosed system is intended to empower surgical robots and navigation systems for spinal surgeries to increase procedure efficiency and reduce surgery duration by providing information on implant types and sizes, along with expected insertion angles.

### SUMMARY

The invention is defined in the appended claims. The present invention is directed to a surgical guidance system for computer assisted navigation during spinal surgery. The surgical guidance system is operative to obtain feedback data provided by distributed networked computers for each of a plurality of prior patients who have undergone spinal surgery. The feedback data characterizes spinal geometric structures of the prior patient, characterizes a surgical procedure performed on the prior patient, characterizes an implant device that was surgically implanted into the prior patient's spine, and characterizes the prior patient's surgical outcome. The surgical guidance system is operative to train a machine learning model based on the feedback data. The surgical guidance system is operative to obtain pre-operative data from one of the distributed network computers characterizing spinal geometric structures of a candidate patient for planned surgery, generate a surgical plan for the candidate patient based on processing the pre-operative data through the machine learning model, and provide at least a portion of the surgical plan to a display device for visual review by a user.

According to the claimed invention, the surgical system includes a surgical guidance system, a tracking system, and at least one controller. The surgical guidance system is operative to obtain feedback data provided by distributed networked computers for each of a plurality of prior patients who have undergone spinal surgery, train a machine learning model based on the feedback data, obtain pre-operative data from one of the distributed network computers characterizing spinal geometric structures of a candidate patient for planned surgery, and generate a surgical plan for the candidate patient based on processing the preoperative data through the machine learning model, where the surgical plan identifies type and dimension sizing of a spinal implant device for surgical implantation in the spine of the candidate patient and identifies a planned trajectory for implantation of the spinal implant device. The tracking system is operative to determine a present pose of a surgical tool being used to implant the spinal implant device in the spine of the candidate patient. The at least one controller is operative to generate navigation information based on comparison of the present pose of the surgical tool and the planned trajectory, where the navigation information indicates how the surgical tool needs to be posed to be aligned with the planned trajectory, and provide the navigation information to a display device.

The claimed surgical system further includes an extended reality (XR) headset including the display device. The at least one controller is operative to generate a graphical representation of the navigation information that is provided to the display device of the XR headset to guide operator movement of the surgical tool along the planned trajectory.

The surgical system according to the claimed invention further includes a surgical robot including a robot base, a robot arm connected to the robot base, where the robot arm is configured to connect to an end effector which guides movement of the surgical tool, and at least one motor operatively connected to move the robot arm relative to the robot base. The at least one controller is connected to the at least one motor and operative to determine a pose of the end effector relative to a planned pose of the end effector while guiding movement of the surgical tool along the planned trajectory during implantation of the spinal implant device, and generate navigation information based on comparison of the planned pose and the determined pose of the end effector, wherein the navigation information indicates where the end effector needs to be moved to become aligned with the planned pose so the surgical tool will be guided by the end effector along the planned trajectory toward the patient.

Other surgical systems, surgical guidance systems, and corresponding methods and computer program products will be or become apparent to one with skill in the art upon review of the following drawings and detailed description. It is intended that all such surgical systems included within this description, be within the scope of the present invention, as long as they fall within the scope of the accompanying claims. Moreover, it is intended that all embodiments disclosed herein can be implemented separately or combined in any way and/or combination.

### BRIEF DESCRIPTION OF THE DRAWINGS

The accompanying drawings, which are included to provide a further understanding of the disclosure and are incorporated in a constitute a part of this application, illustrate certain non-limiting embodiments of inventive concepts. In the drawings:
Figure 1 illustrates an embodiment of a surgical system according to some embodiments of the present disclosure;
Figure 2 illustrates a surgical robot component of the surgical system of Figure 1 according to some embodiments of the present disclosure;
Figure 3A illustrates a camera tracking system component of the surgical system of Figure 1 according to some embodiments of the present disclosure;
Figures 3B and 3C illustrate a front view and isometric view of another camera tracking system component which may be used with the surgical system of Figure 1 according to some embodiments of the present disclosure;
Figure 4 illustrates an embodiment of an end effector that is connectable to a robot arm and configured according to some embodiments of the present disclosure;
Figure 5 illustrates a medical operation in which a surgical robot and a camera system are disposed around a patient;
Figure 6 illustrates a block diagram view of the components of the surgical system of Figure 5 being used for a medical operation;
Figure 7 illustrates various display screens that may be displayed on the display of Figures 5 and 6 when using a navigation function of the surgical system;
Figure 8 illustrates a block diagram of some electrical components of a surgical robot according to some embodiments of the present disclosure;
Figure 9 illustrates a block diagram of components of a surgical system that includes imaging devices connected to a computer platform which can be operationally connected to a camera tracking system and/or surgical robot according to some embodiments of the present disclosure;
Figure 10 illustrates electrical components of an XR headset that can be operatively connected to a computer platform, to one or more of the imaging devices, and/or to a surgical robot in accordance with various embodiments of the present disclosure;
Figure 11 illustrates an example view through an XR headset for providing navigation assistance to a user who is manipulating a surgical tool during a medical procedure in accordance with some embodiments of the present disclosure;
Figure 12 illustrates a navigated surgery workflow which uses a surgical guidance system 1220 configured in accordance with some embodiments;
Figure 13 illustrates a block diagram of the surgical guidance system with associated data flows during the pre-operative, intra-operative, and post-operative stages, and shows surgical guidance being provided to user displays and to a robot surgery system in accordance with some embodiments;
Figures 14 and 15 illustrate a planned surgical procedure which includes using a selected surgical tool which is adapted for insertion of a selected spacer into a planned pose within a disc gap of a spine in accordance with some embodiments; and
Figure 16 illustrates a flowchart of operations that can be performed by a surgical system which may include a surgical guidance system, a computer platform, a camera tracking system component, and a surgical robot 4 in accordance with some embodiments.

### DETAILED DESCRIPTION

Inventive concepts will now be described more fully hereinafter with reference to the accompanying drawings, in which examples of embodiments of inventive concepts are shown. Inventive concepts may, however, be embodied in many different forms and should not be construed as limited to the embodiments set forth herein. Rather, these embodiments are provided so that this disclosure will be thorough and complete, and will fully convey the scope of various present inventive concepts to those skilled in the art, as defined by the appended claims. It should also be noted that these embodiments are not mutually exclusive. Components from one embodiment may be tacitly assumed to be present or used in another embodiment.

Various embodiments disclosed herein are directed to improvements in operation of a surgical system providing navigated guidance when planning for and performing spinal surgery. A machine learning (ML) guidance system provides patient customized guidance during preoperative stage planning, intra-operative stage surgical procedures, and post-operative stage assessment. A central database stores data that can be obtained in each of the stages across all patients who have previously used or are currently using the ML guidance system. The ML system is trained over time based on data from the central database so that the patient customized guidance provides improved surgical outcomes.

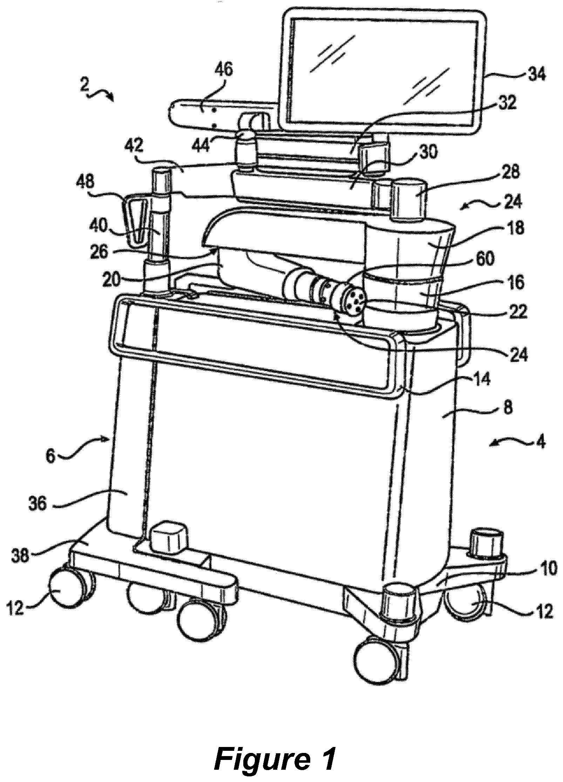

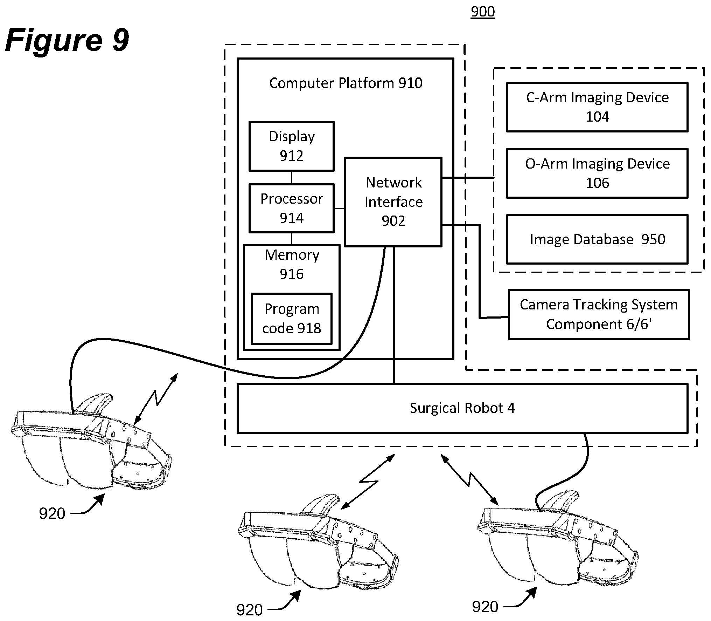

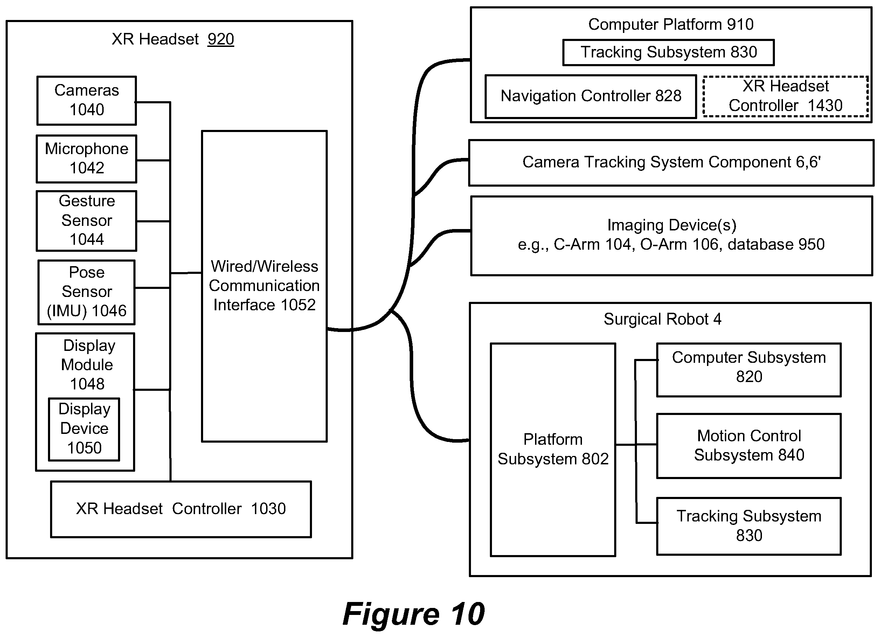

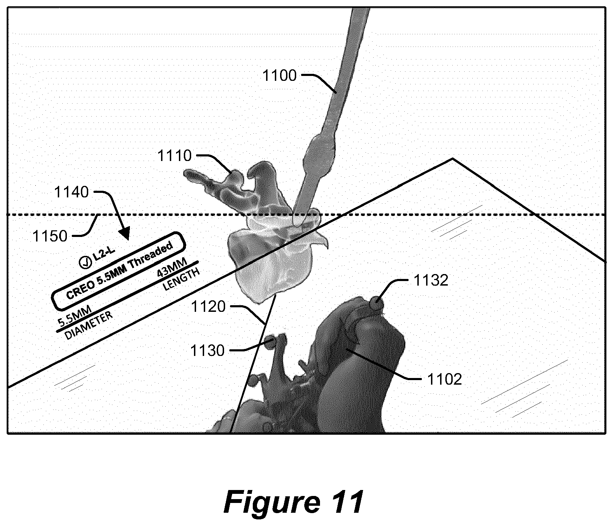

Figure 1 illustrates an embodiment of a surgical system 2 according to some embodiments of the present disclosure. Prior to performance of an orthopedic or other surgical procedure, a three-dimensional ("3D") image scan may be taken of a planned surgical area of a patient using, e.g., the C-Arm imaging device 104 of Fig. 10 or O-Arm imaging device 106 of Fig. 11, or from another medical imaging device such as a computed tomography (CT) image or MRI. This scan can be taken pre-operatively (e.g. few weeks before procedure, most common) or intra-operatively. However, any known 3D or 2D image scan may be used in accordance with various embodiments of the surgical system 2. The image scan is sent to a computer platform in communication with the surgical system 2, such as the computer platform 910 of the surgical system 900 (Fig. 9) which may include the camera tracking system component 6, the surgical robot 4 (e.g., robot 2 in Fig. 1), imaging devices (e.g., C-Arm 104, O-Arm 106, etc.), and an image database 950 for storing image scans of patients. A surgeon reviewing the image scan(s) on a display device of the computer platform 910 (Fig. 9) generates a surgical plan defining a target pose for a surgical tool to be used during a surgical procedure on an anatomical structure of the patient. Example surgical tools, also referred to as tools, can include, without limitation, drills, screw drivers, saws, retractors, and implants such as a screws, spacers, interbody fusion devices, plates, rods, etc. In some embodiments, the surgical plan defining the target plane is planned on the 3D image scan displayed on a display device.

As used herein, the term "pose" refers to the position and/or the rotational angle of one object (e.g., dynamic reference array, end effector, surgical tool, anatomical structure, etc.) relative to another object and/or to a defined coordinate system. A pose may therefore be defined based on only the multidimensional position of one object relative to another object and/or to a defined coordinate system, only on the multidimensional rotational angles of the object relative to another object and/or to a defined coordinate system, or on a combination of the multidimensional position and the multidimensional rotational angles. The term "pose" therefore is used to refer to position, rotational angle, or combination thereof.

The surgical system 2 of Figure 1 can assist surgeons during medical procedures by, for example, holding tools, aligning tools, using tools, guiding tools, and/or positioning tools for use. In some embodiments, surgical system 2 includes a surgical robot 4 and a camera tracking system component 6. The ability to mechanically couple surgical robot 4 and camera tracking system component 6 can allow for surgical system 2 to maneuver and move as a single unit, and allow surgical system 2 to have a small footprint in an area, allow easier movement through narrow passages and around turns, and allow storage within a smaller area.

An orthopedic surgical procedure may begin with the surgical system 2 moving from medical storage to a medical procedure room. The surgical system 2 may be maneuvered through doorways, halls, and elevators to reach a medical procedure room. Within the room, the surgical system 2 may be physically separated into two separate and distinct systems, the surgical robot 4 and the camera tracking system 6. Surgical robot 4 may be positioned adjacent the patient at any suitable location to properly assist medical personnel. Camera tracking system component 6 may be positioned at the base of the patient, at patient shoulders or any other location suitable to track the present pose and movement of the pose of tracks portions of the surgical robot 4 and the patient. Surgical robot 4 and Camera tracking system component 6 may be powered by an onboard power source and/or plugged into an external wall outlet.

Surgical robot 4 may be used to assist a surgeon by holding and/or using tools during a medical procedure. To properly utilize and hold tools, surgical robot 4 may rely on a plurality of motors, computers, and/or actuators to function properly. Illustrated in Figure 1, robot body 8 may act as the structure in which the plurality of motors, computers, and/or actuators may be secured within surgical robot 4. Robot body 8 may also provide support for robot telescoping support arm 16. The size of robot body 8 may provide a solid platform supporting attached components, and may house, conceal, and protect the plurality of motors, computers, and/or actuators that may operate attached components.

Robot base 10 may act as a lower support for surgical robot 4. In some embodiments, robot base 10 may support robot body 8 and may attach robot body 8 to a plurality of powered wheels 12. This attachment to wheels may allow robot body 8 to move in space efficiently. Robot base 10 may run the length and width of robot body 8. Robot base 10 may be about two inches to about 10 inches tall. Robot base 10 may cover, protect, and support powered wheels 12.

In some embodiments, as illustrated in Figure 1, at least one powered wheel 12 may be attached to robot base 10. Powered wheels 12 may attach to robot base 10 at any location. Each individual powered wheel 12 may rotate about a vertical axis in any direction. A motor may be disposed above, within, or adjacent to powered wheel 12. This motor may allow for surgical system 2 to maneuver into any location and stabilize and/or level surgical system 2. A rod, located within or adjacent to powered wheel 12, may be pressed into a surface by the motor. The rod, not pictured, may be made of any suitable metal to lift surgical system 2. The rod may lift powered wheel 10, which may lift surgical system 2, to any height required to level or otherwise fix the orientation of the surgical system 2 in relation to a patient. The weight of surgical system 2, supported through small contact areas by the rod on each wheel, prevents surgical system 2 from moving during a medical procedure. This rigid positioning may prevent objects and/or people from moving surgical system 2 by accident.

Moving surgical system 2 may be facilitated using robot railing 14. Robot railing 14 provides a person with the ability to move surgical system 2 without grasping robot body 8. As illustrated in Figure 1, robot railing 14 may run the length of robot body 8, shorter than robot body 8, and/or may run longer the length of robot body 8. Robot railing 14 may further provide protection to robot body 8, preventing objects and or personnel from touching, hitting, or bumping into robot body 8.

Robot body 8 may provide support for a Selective Compliance Articulated Robot Arm, hereafter referred to as a "SCARA." A SCARA 24 may be beneficial to use within the surgical system 2 due to the repeatability and compactness of the robotic arm. The compactness of a SCARA may provide additional space within a medical procedure, which may allow medical professionals to perform medical procedures free of excess clutter and confining areas. SCARA 24 may comprise robot telescoping support 16, robot support arm 18, and/or robot arm 20. Robot telescoping support 16 may be disposed along robot body 8. As illustrated in Figure 1, robot telescoping support 16 may provide support for the SCARA 24 and display 34. In some embodiments, robot telescoping support 16 may extend and contract in a vertical direction. The body of robot telescoping support 16 may be any width and/or height configured to support the stress and weight placed upon it.

In some embodiments, medical personnel may move SCARA 24 through a command submitted by the medical personnel. The command may originate from input received on display 34, a tablet, and/or an XR headset (e.g., headset 920 in Fig. 9) as will be explained in further detail below. The XR headset may eliminate the need for medical personnel to refer to any other display such as the display 34 or a tablet, which enables the SCARA 24 to be configured without the display 34 and/or the tablet. The command may be generated by the depression of a switch and/or the depression of a plurality of switches, and/or may be generated based on a hand gesture command and/or voice command that is sensed by the XR headset as will be explained in further detail below.

An activation assembly 60 may include a switch and/or a plurality of switches. The activation assembly 60 may be operable to transmit a move command to the SCARA 24 allowing an operator to manually manipulate the SCARA 24. When the switch, or plurality of switches, is depressed the medical personnel may have the ability to move SCARA 24 through applied hand movements. Alternatively or additionally, an operator may control movement of the SCARA 24 through hand gesture commands and/or voice commands that are sensed by the XR headset as will be explained in further detail below. Additionally, when the SCARA 24 is not receiving a command to move, the SCARA 24 may lock in place to prevent accidental movement by personnel and/or other objects. By locking in place, the SCARA 24 provides a solid platform through which the end effector 26 can guide a surgical tool during a medical procedure.

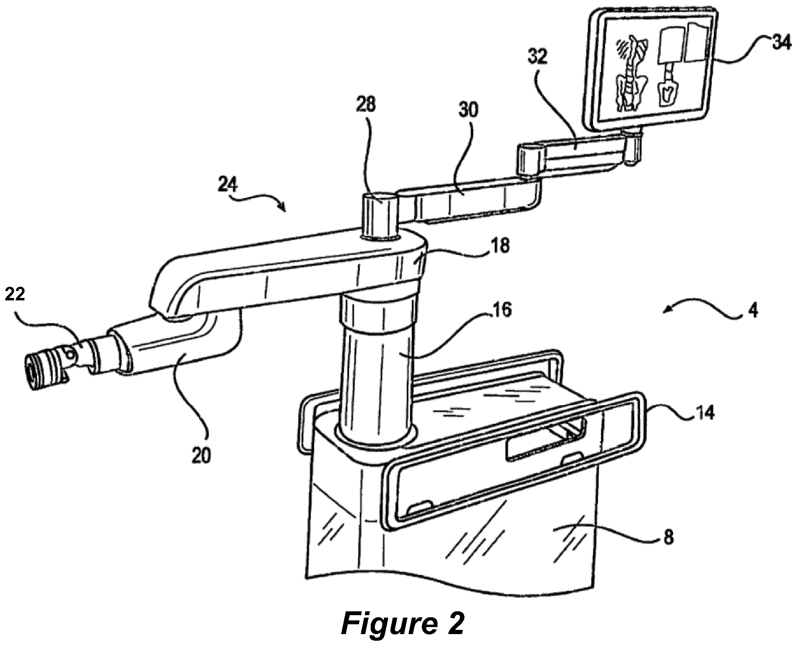

Robot support arm 18 can be connected to robot telescoping support 16 by various mechanisms. In some embodiments, best seen in Figures 1 and 2, robot support arm 18 rotates in any direction in regard to robot telescoping support 16. Robot support arm 18 may rotate three hundred and sixty degrees around robot telescoping support 16. Robot arm 20 may connect to robot support arm 18 at any suitable location and by various mechanisms that enable rotation in any direction relative to robot support arm 18. In one embodiment, the robot arm 20 can rotate three hundred and sixty degrees relative to the robot support arm 18. This free rotation allows an operator to position robot arm 20 according to a surgical plan.

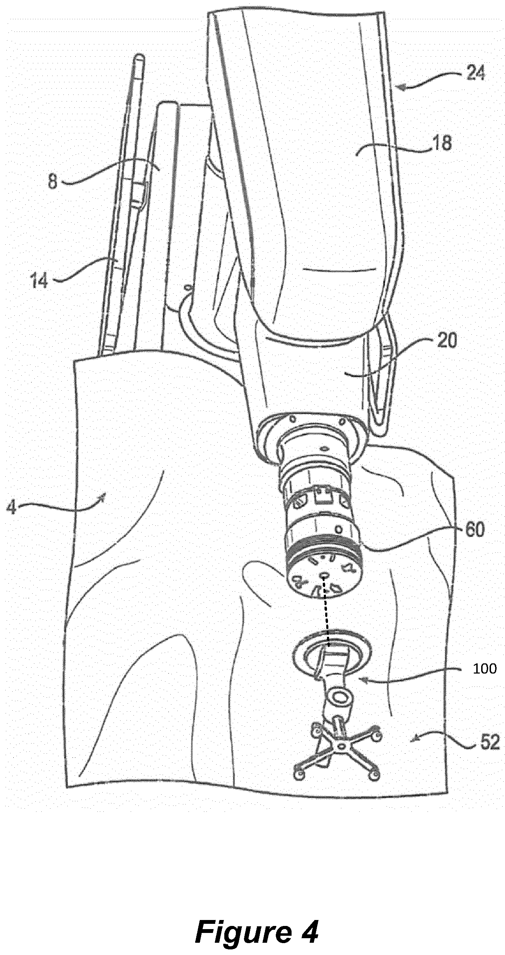

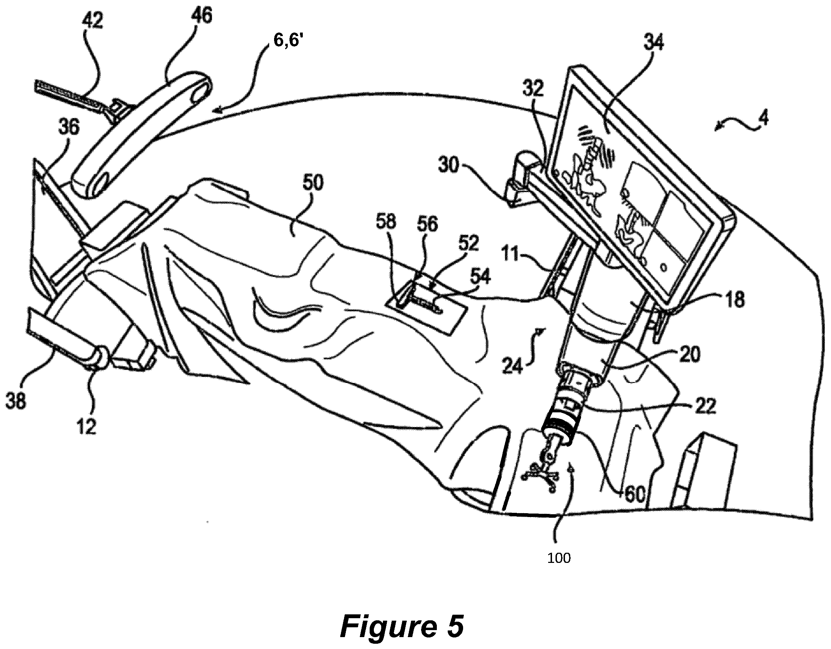

The passive end effector 100 in Figures 4 and 5 may attach to robot arm 20 in any suitable location, such as to an end effector coupler 22 of the robot arm 20 positioned by the surgical robot 4. The illustrated passive end effector 100 includes a guide tube which is positioned by a navigation system controlling the robot arm 20 to be posed relative to a target patient anatomical structure (e.g., vertebrae) according to a surgical plan. The surgeon can then inserted tools through the guide tube which maintains a proper trajectory of the tool relative to a target patient anatomical structure.

The passive end effector 100 has an attached dynamic reference array 52, or a dynamic reference array formed on it. Dynamic reference arrays, also referred to as "DRAs" and "reference arrays" herein, can be rigid bodies, markers, or other indicia which are attached or formed on one or more XR headsets being worn by personnel in the operating room, the end effector, the surgical robot, a surgical tool in a navigated surgical procedure, and an anatomical structure (e.g., bone) of a patient. The computer platform 910 in combination with the camera tracking system component 6 or other 3D localization system are configured to track in real-time the pose (e.g., positions and rotational orientations) of the DRA. The DRA can include fiducials, such as the illustrated arrangement of balls. This tracking of 3D coordinates of the DRA can allow the surgical system 2 to determine the pose of the DRA in any multidimensional space in relation to the target anatomical structure of the patient 50 in Figure 5.

As illustrated in Figure 1, a light indicator 28 may be positioned on top of the SCARA 24. Light indicator 28 may illuminate as any type of light to indicate "conditions" in which surgical system 2 is currently operating. In some embodiments, the light may be produced by LED bulbs, which may form a ring around light indicator 28. Light indicator 28 may comprise a fully permeable material that can let light shine through the entirety of light indicator 28. Light indicator 28 may be attached to lower display support 30. Lower display support 30, as illustrated in Figure 2 may allow an operator to maneuver display 34 to any suitable location. Lower display support 30 may attach to light indicator 28 by any suitable mechanism. In some embodiments, lower display support 30 may rotate about light indicator 28 or be rigidly attached thereto. Upper display support 32 may attach to lower display support 30 by any suitable mechanism.

In some embodiments, a tablet may be used in conjunction with display 34 and/or without display 34. The tablet may be disposed on upper display support 32, in place of display 34, and may be removable from upper display support 32 during a medical operation. In addition the tablet may communicate with display 34. The tablet may be able to connect to surgical robot 4 by any suitable wireless and/or wired connection. In some embodiments, the tablet may be able to program and/or control surgical system 2 during a medical operation. When controlling surgical system 2 with the tablet, all input and output commands may be duplicated on display 34. The use of a tablet may allow an operator to manipulate surgical robot 4 without having to move around patient 50 and/or to surgical robot 4.

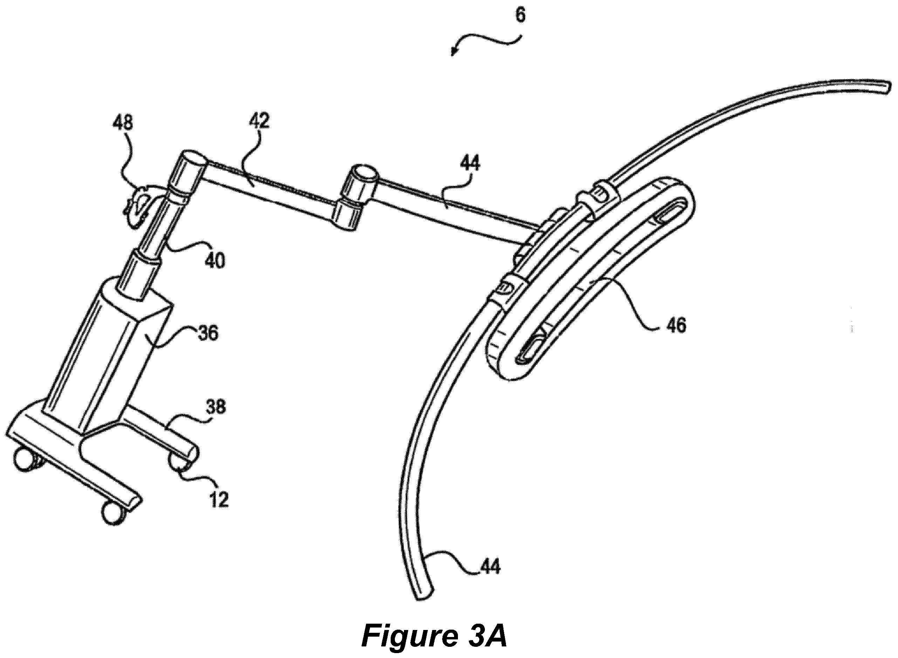

As illustrated in Figures 3A and 5, camera tracking system component 6 works in conjunction with surgical robot 4 through wired or wireless communication networks. Referring to Figures 1, 3 and 5, camera tracking system component 6 can include some similar components to the surgical robot 4. For example, camera body 36 may provide the functionality found in robot body 8. Robot body 8 may provide an auxiliary tracking bar upon which cameras 46 are mounted. The structure within robot body 8 may also provide support for the electronics, communication devices, and power supplies used to operate camera tracking system component 6. Camera body 36 may be made of the same material as robot body 8. Camera tracking system component 6 may communicate directly to an XR headset, tablet and/or display 34 by a wireless and/or wired network to enable the XR headset, tablet and/or display 34 to control the functions of camera tracking system component 6.

Camera body 36 is supported by camera base 38. Camera base 38 may function as robot base 10. In the embodiment of Figure 1, camera base 38 may be wider than robot base 10. The width of camera base 38 may allow for camera tracking system component 6 to connect with surgical robot 4. As illustrated in Figure 1, the width of camera base 38 may be large enough to fit outside robot base 10. When camera tracking system component 6 and surgical robot 4 are connected, the additional width of camera base 38 may allow surgical system 2 additional maneuverability and support for surgical system 2.

As with robot base 10, a plurality of powered wheels 12 may attach to camera base 38. Powered wheel 12 may allow camera tracking system component 6 to stabilize and level or set fixed orientation in regards to patient 50, similar to the operation of robot base 10 and powered wheels 12. This stabilization may prevent camera tracking system component 6 from moving during a medical procedure and may keep cameras 46 on the auxiliary tracking bar from losing track of a DRA connected to an XR headset and/or the surgical robot 4, and/or losing track of one or more DRAs 52 connected to an anatomical structure 54 and/or tool 58 within a designated area 56 as shown in Figures 3A and 5. This stability and maintenance of tracking enhances the ability of surgical robot 4 to operate effectively with camera tracking system component 6. Additionally, the wide camera base 38 may provide additional support to camera tracking system component 6. Specifically, a wide camera base 38 may prevent camera tracking system component 6 from tipping over when cameras 46 is disposed over a patient, as illustrated in Figures 3A and 5.

Camera telescoping support 40 may support cameras 46 on the auxiliary tracking bar. In some embodiments, telescoping support 40 moves cameras 46 higher or lower in the vertical direction. Camera handle 48 may be attached to camera telescoping support 40 at any suitable location and configured to allow an operator to move camera tracking system component 6 into a planned position before a medical operation. In some embodiments, camera handle 48 is used to lower and raise camera telescoping support 40. Camera handle 48 may perform the raising and lowering of camera telescoping support 40 through the depression of a button, switch, lever, and/or any combination thereof.

Lower camera support arm 42 may attach to camera telescoping support 40 at any suitable location, in embodiments, as illustrated in Figure 1, lower camera support arm 42 may rotate three hundred and sixty degrees around telescoping support 40. This free rotation may allow an operator to position cameras 46 in any suitable location. Lower camera support arm 42 may connect to telescoping support 40 by any suitable mechanism. Lower camera support arm 42 may be used to provide support for cameras 46. Cameras 46 may be attached to lower camera support arm 42 by any suitable mechanism. Cameras 46 may pivot in any direction at the attachment area between cameras 46 and lower camera support arm 42. In embodiments a curved rail 44 may be disposed on lower camera support arm 42.

Curved rail 44 may be disposed at any suitable location on lower camera support arm 42. As illustrated in Figure 3A, curved rail 44 may attach to lower camera support arm 42 by any suitable mechanism. Curved rail 44 may be of any suitable shape, a suitable shape may be a crescent, circular, oval, elliptical, and/or any combination thereof. Cameras 46 may be moveably disposed along curved rail 44. Cameras 46 may attach to curved rail 44 by, for example, rollers, brackets, braces, motors, and/or any combination thereof. Motors and rollers, not illustrated, may be used to move cameras 46 along curved rail 44. As illustrated in Figure 3A, during a medical procedure, if an object prevents cameras 46 from viewing one or more DRAs being tracked, the motors may responsively move cameras 46 along curved rail 44. This motorized movement may allow cameras 46 to move to a new position that is no longer obstructed by the object without moving camera tracking system component 6. While cameras 46 is obstructed from viewing one or more tracked DRAs, camera tracking system component 6 may send a stop signal to a surgical robot 4, XR headset, display 34, and/or a tablet. The stop signal may prevent SCARA 24 from moving until cameras 46 has reacquired tracked DRAs 52 and/or can warn an operator wearing the XR headset and/or viewing the display 34 and/or the tablet. This SCARA 24 can be configured to respond to receipt of a stop signal by stopping further movement of the base and/or end effector coupler 22 until the camera tracking system can resume tracking of DRAs.

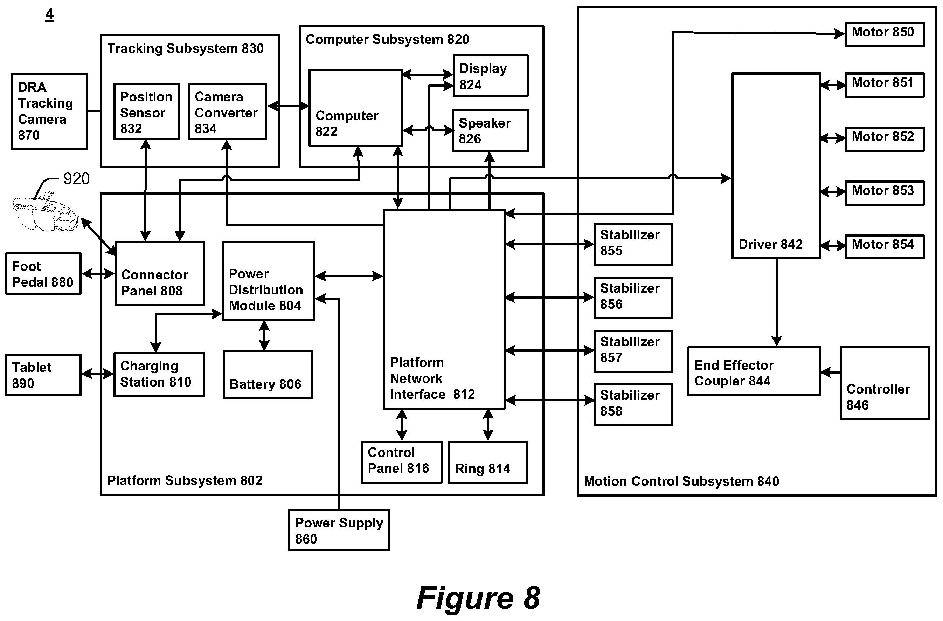

The end effector coupler 22 can include a load cell interposed between a saddle join and a connected passive end effector. The load cell may be any suitable instrument used to detect and measure forces. In some examples, the load cell may be a six axis load cell, a threeaxis load cell or a uniaxial load cell. The load cell may be used to track the force applied to end effector coupler 22. In some embodiments the load cell may communicate with a plurality of motors 850, 851, 852, 853, and/or 854. As the load cell senses force, information as to the amount of force applied may be distributed to a controller 846 (Fig. 8). Controller 846 may take the force information from the load cell and process it with a switch algorithm. The switch algorithm is used by the controller 846 to control a motor driver 842. The motor driver 842 controls operation of one or more of the motors. Motor driver 842 may direct a specific motor to produce, for example, an equal amount of force measured by load cell through the motor. In some embodiments, the force produced may come from a plurality of motors, e.g., 850-854, as directed by controller 846. Additionally, motor driver 842 may receive input from controller 846. Controller 846 may receive information from load cell as to the direction of force sensed by load cell. Controller 846 may process this information using a motion controller algorithm. The algorithm may be used to provide information to specific motor drivers 842. To replicate the direction of force, controller 846 may activate and/or deactivate certain motor drivers 842. Controller 846 may control one or more motors, e.g. one or more of 850-854, to induce motion of passive end effector 100 in the direction of force sensed by load cell. This force-controlled motion may allow an operator to move SCARA 24 and passive end effector 100 effortlessly and/or with very little resistance. Movement of passive end effector 100 can be performed to position passive end effector 100 in any suitable pose (i.e., location and angular orientation relative to defined three-dimensional (3D) orthogonal reference axes) for use by medical personnel.

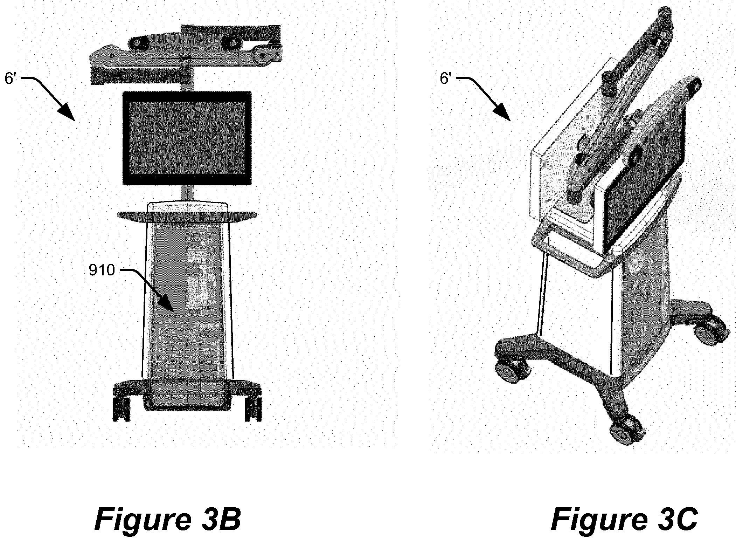

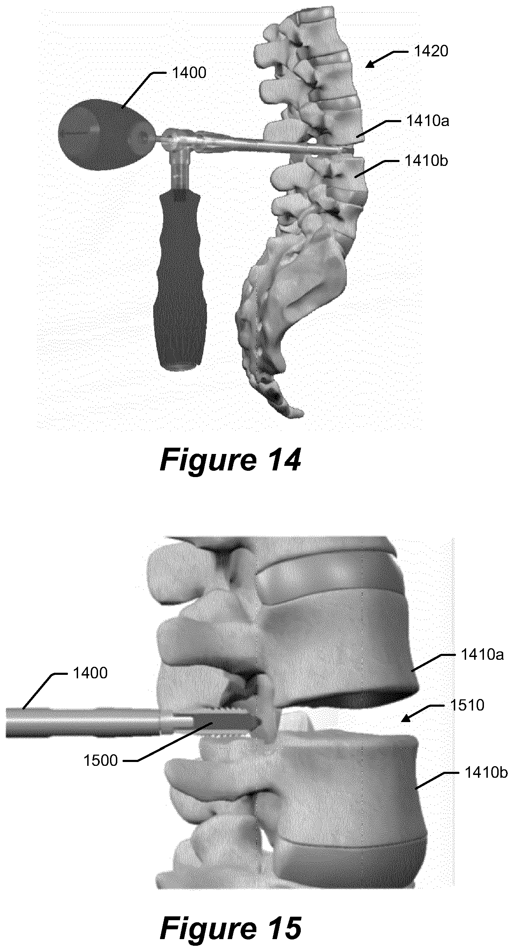

Figures 3B and 3C illustrate a front view and isometric view of another camera tracking system component 6' which may be used with the surgical system of Figure 1 or may be used independent of a surgical robot. For example, the camera tracking system component 6' may be used for providing navigated surgery without use of robotic guidance. One of the differences between the camera tracking system component 6' of Figures 3B and 3C and the camera tracking system component 6 of Figure 3A, is that the camera tracking system component 6' of Figures 3B and 3C includes a housing that transports the computer platform 910. The computer platform 910 can be configured to perform camera tracking operations to track DRAs, perform navigated surgery operations that provide surgical navigation information to a display device, e.g., XR headset and/or other display device, and perform other computational operations disclosed herein. The computer platform 910 can therefore include a navigation computer, such as one or more of the navigation computers of Figures 14.

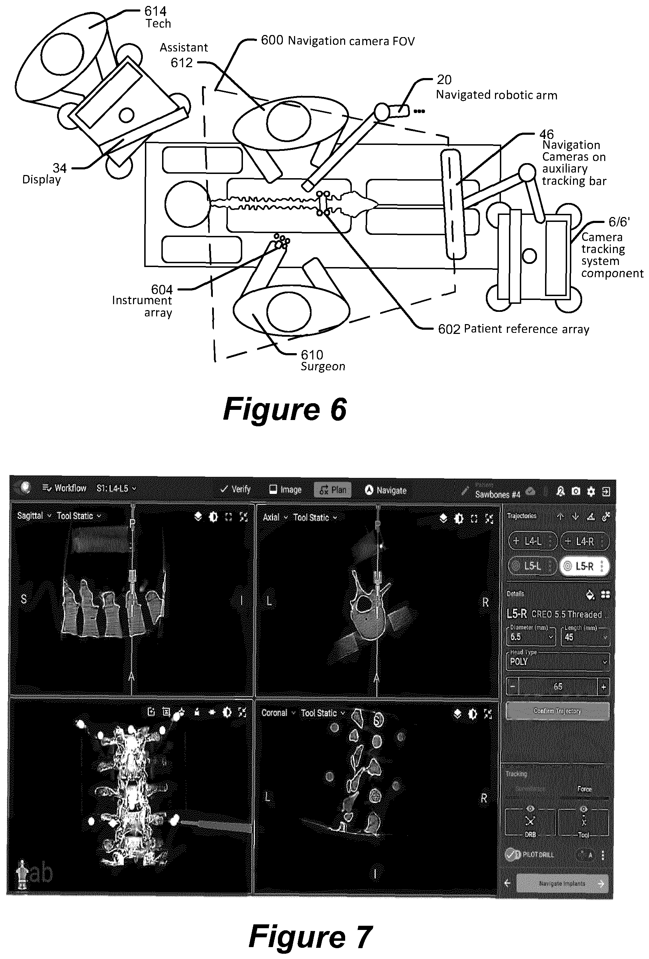

Figure 6 illustrates a block diagram view of the components of the surgical system of Figure 5 used for the medical operation. Referring to Figure 6, the tracking cameras 46 on the auxiliary tracking bar has a navigation field-of-view 600 in which the pose (e.g., position and orientation) of the reference array 602 attached to the patient, the reference array 604 attached to the surgical instrument, and the robot arm 20 are tracked. The tracking cameras 46 may be part of the camera tracking system component 6' of Figures 3B and 3C, which includes the computer platform 910 configured to perform the operations described below. The reference arrays enable tracking by reflecting light in known patterns, which are decoded to determine their respective poses by the tracking subsystem of the surgical robot 4. If the lineof-sight between the patient reference array 602 and the tracking cameras 46 in the auxiliary tracking bar is blocked (for example, by a medical personnel, instrument, etc.), further navigation of the surgical instrument may not be able to be performed and a responsive notification may temporarily halt further movement of the robot arm 20 and surgical robot 4, display a warning on the display 34, and/or provide an audible warning to medical personnel. The display 34 is accessible to the surgeon 610 and assistant 612 but viewing requires a head to be turned away from the patient and for eye focus to be changed to a different distance and location. The navigation software may be controlled by a tech personnel 614 based on vocal instructions from the surgeon.

Figure 7 illustrates various display screens that may be displayed on the display 34 of Figures 5 and 6 by the surgical robot 4 when using a navigation function of the surgical system 2. The display screens can include, without limitation, patient radiographs with overlaid graphical representations of models of instruments that are positioned in the display screens relative to the anatomical structure based on a developed surgical plan and/or based on poses of tracked reference arrays, various user selectable menus for controlling different stages of the surgical procedure and dimension parameters of a virtually projected implant (e.g. length, width, and/or diameter).

For navigated surgery, various processing components (e.g., computer platform 910) and associated software described below are provided that enable pre-operatively planning of a surgical procedure, e.g., implant placement, and electronic transfer of the plan to computer platform 910 to provide navigation information to one or more users during the planned surgical procedure.

For robotic navigation, various processing components (e.g., computer platform 910) and associated software described below are provided that enable pre-operatively planning of a surgical procedure, e.g., implant placement, and electronic transfer of the plan to the surgical robot 4. The surgical robot 4 uses the plan to guide the robot arm 20 and connected end effector 26 to provide a target pose for a surgical tool relative to a patient anatomical structure for a step of the planned surgical procedure.

Various embodiments below are directed to using one or more XR headsets that can be worn by the surgeon 610, the assistant 612, and/or other medical personnel to provide an improved user interface for receiving information from and/or providing control commands to the surgical robot, the camera tracking system component 6/6', and/or other medical equipment in the operating room.

Figure 8 illustrates a block diagram of some electrical components of the surgical robot 4 according to some embodiments of the present disclosure. Referring to Figure 8, a load cell (not shown) may be configured to track force applied to end effector coupler 22. In some embodiments the load cell may communicate with a plurality of motors 850, 851, 852, 853, and/or 854. As load cell senses force, information as to the amount of force applied may be distributed from a switch array and/or a plurality of switch arrays to a controller 846. Controller 846 may take the force information from load cell and process it with a switch algorithm. The switch algorithm is used by the controller 846 to control a motor driver 842. The motor driver 842 controls operation of one or more of the motors 850, 851, 852, 853, and 854. Motor driver 842 may direct a specific motor to produce, for example, an equal amount of force measured by load cell through the motor. In some embodiments, the force produced may come from a plurality of motors, e.g., 850-854, as directed by controller 846. Additionally, motor driver 842 may receive input from controller 846. Controller 846 may receive information from load cell as to the direction of force sensed by load cell. Controller 846 may process this information using a motion controller algorithm. The algorithm may be used to provide information to specific motor drivers 842. To replicate the direction of force, controller 846 may activate and/or deactivate certain motor drivers 842. Controller 846 may control one or more motors, e.g. one or more of 850-854, to induce motion of end effector 26 in the direction of force sensed by load cell. This force-controlled motion may allow an operator to move SCARA 24 and end effector 26 effortlessly and/or with very little resistance. Movement of end effector 26 can be performed to position end effector 26 in any suitable pose (i.e., location and angular orientation relative to defined three-dimensional (3D) orthogonal reference axes) for use by medical personnel.

Activation assembly 60, best illustrated in Figure 5, may form of a bracelet that wraps around end effector coupler 22. The activation assembly 60 may be located on any part of SCARA 24, any part of end effector coupler 22, may be worn by medical personnel (and communicate wirelessly), and/or any combination thereof. Activation assembly 60 may comprise of a primary button and a secondary button.

Depressing primary button may allow an operator to move SCARA 24 and end effector coupler 22. According to one embodiment, once set in place, SCARA 24 and end effector coupler 22 may not move until an operator programs surgical robot 4 to move SCARA 24 and end effector coupler 22, or is moved using primary button. In some examples, it may require the depression of at least two non-adjacent primary activation switches before SCARA 24 and end effector coupler 22 will respond to operator commands. Depression of at least two primary activation switches may prevent the accidental movement of SCARA 24 and end effector coupler 22 during a medical procedure.

Activated by primary button, load cell may measure the force magnitude and/or direction exerted upon end effector coupler 22 by an operator, i.e. medical personnel. This information may be transferred to one or more motors, e.g. one or more of 850-854, within SCARA 24 that may be used to move SCARA 24 and end effector coupler 22. Information as to the magnitude and direction of force measured by load cell may cause the one or more motors, e.g. one or more of 850-854, to move SCARA 24 and end effector coupler 22 in the same direction as sensed by the load cell. This force-controlled movement may allow the operator to move SCARA 24 and end effector coupler 22 easily and without large amounts of exertion due to the motors moving SCARA 24 and end effector coupler 22 at the same time the operator is moving SCARA 24 and end effector coupler 22.

In some examples, a secondary button may be used by an operator as a "selection" device. During a medical operation, surgical robot 4 may notify medical personnel to certain conditions by the XR headset(s) 920, display 34 and/or light indicator 28. The XR headset(s) 920 are each configured to display images on a see-through display screen to form an extended reality image that is overlaid on real-world objects viewable through the see-through display screen. Medical personnel may be prompted by surgical robot 4 to select a function, mode, and/or asses the condition of surgical system 2. Depressing secondary button a single time may activate certain functions, modes, and/or acknowledge information communicated to medical personnel through the XR headset(s) 920, display 34 and/or light indicator 28. Additionally, depressing the secondary button multiple times in rapid succession may activate additional functions, modes, and/or select information communicated to medical personnel through the XR headset(s) 920, display 34 and/or light indicator 28.

With further reference to Figure 8, electrical components of the surgical robot 4 include platform subsystem 802, computer subsystem 820, motion control subsystem 840, and tracking subsystem 830. Platform subsystem 802 includes battery 806, power distribution module 804, connector panel 808, and charging station 810. Computer subsystem 820 includes computer 822, display 824, and speaker 826. Motion control subsystem 840 includes driver circuit 842, motors 850, 851, 852, 853, 854, stabilizers 855, 856, 857, 858, end effector connector 844, and controller 846. Tracking subsystem 830 includes position sensor 832 and camera converter 834. Surgical robot 4 may also include a removable foot pedal 880 and removable tablet computer 890.

Input power is supplied to surgical robot 4 via a power source which may be provided to power distribution module 804. Power distribution module 804 receives input power and is configured to generate different power supply voltages that are provided to other modules, components, and subsystems of surgical robot 4. Power distribution module 804 may be configured to provide different voltage supplies to connector panel 808, which may be provided to other components such as computer 822, display 824, speaker 826, driver 842 to, for example, power motors 850-854 and end effector coupler 844, and provided to camera converter 834 and other components for surgical robot 4. Power distribution module 804 may also be connected to battery 806, which serves as temporary power source in the event that power distribution module 804 does not receive power from an input power. At other times, power distribution module 804 may serve to charge battery 806.

Connector panel 808 may serve to connect different devices and components to surgical robot 4 and/or associated components and modules. Connector panel 808 may contain one or more ports that receive lines or connections from different components. For example, connector panel 808 may have a ground terminal port that may ground surgical robot 4 to other equipment, a port to connect foot pedal 880, a port to connect to tracking subsystem 830, which may include position sensor 832, camera converter 834, and DRA tracking cameras 870. Connector panel 808 may also include other ports to allow USB, Ethernet, HDMI communications to other components, such as computer 822. In accordance with some embodiments, the connector panel 808 can include a wired and/or wireless interface for operatively connecting one or more XR headsets 920 to the tracking subsystem 830 and/or the computer subsystem 820.

Control panel 816 may provide various buttons or indicators that control operation of surgical robot 4 and/or provide information from surgical robot 4 for observation by an operator. For example, control panel 816 may include buttons to power on or off surgical robot 4, lift or lower vertical column 16, and lift or lower stabilizers 855-858 that may be designed to engage casters 12 to lock surgical robot 4 from physically moving. Other buttons may stop surgical robot 4 in the event of an emergency, which may remove all motor power and apply mechanical brakes to stop all motion from occurring. Control panel 816 may also have indicators notifying the operator of certain system conditions such as a line power indicator or status of charge for battery 806. In accordance with some embodiments, one or more XR headsets 920 may communicate, e.g. via the connector panel 808, to control operation of the surgical robot 4 and/or to received and display information generated by surgical robot 4 for observation by persons wearing the XR headsets 920.

Computer 822 of computer subsystem 820 includes an operating system and software to operate assigned functions of surgical robot 4. Computer 822 may receive and process information from other components (for example, tracking subsystem 830, platform subsystem 802, and/or motion control subsystem 840) in order to display information to the operator. Further, computer subsystem 820 may provide output through the speaker 826 for the operator. The speaker may be part of the surgical robot, part of an XR headset 920, or within another component of the surgical system 2. The display 824 may correspond to the display 34 shown in Figures 1 and 2.

Tracking subsystem 830 may include position sensor 832 and camera converter 834. Tracking subsystem 830 may correspond to the camera tracking system component 6 of Figure 3. The DRA tracking cameras 870 operate with the position sensor 832 to determine the pose of DRAs 52. This tracking may be conducted in a manner consistent with the present disclosure including the use of infrared or visible light technology that tracks the location of active or passive elements of DRAs 52, such as LEDs or reflective fiducials (also called markers), respectively.

The location, orientation, and position of structures having these types of markers, such as DRAs 52, is provided to computer 822 and which may be shown to an operator on display 824. For example, as shown in Figures 4 and 5, a surgical saw 1040 having a DRA 52 or which is connected to an end effector coupler 22 having a DRA 52 tracked in this manner (which may be referred to as a navigational space) may be shown to an operator in relation to a three dimensional image of a patient's anatomical structure.

Functional operations of the tracking subsystem 830 and the computer subsystem 820 can be included in the computer platform 910, which can be transported by the camera tracking system component 6' of Figures 3A and 3B. The tracking subsystem 830 can be configured to determine the poses, e.g., location and angular orientation of the tracked DRAs. The computer platform 910 includes a navigation controller that is configured to use the determined poses to provide navigation information to users that guides their movement of tracked tools relative to position-registered patient images and/or tracked anatomical structures during a planned surgical procedure. The computer platform 910 can display information on the display of Figures 3B and 3C and/or to one or more XR headsets 920. The computer platform 910, when used with a surgical robot, can be configured to communicate with the computer subsystem 820 and other subsystems of Figure 8 to control movement of the end effector 26. For example, as will be explained below the computer platform 910 can generate a graphical representation of a patient's anatomical structure, surgical tool, user's hand, etc. with a displayed size, shape, color, and/or pose that is controlled based on the determined pose(s) of one or more the tracked DRAs, and which the graphical representation that is displayed can be dynamically modified to track changes in the determined poses over time.

Motion control subsystem 840 may be configured to physically move vertical column 16, upper arm 18, lower arm 20, or rotate end effector coupler 22. The physical movement may be conducted through the use of one or more motors 850-854. For example, motor 850 may be configured to vertically lift or lower vertical column 16. Motor 851 may be configured to laterally move upper arm 18 around a point of engagement with vertical column 16 as shown in Figure 2. Motor 852 may be configured to laterally move lower arm 20 around a point of engagement with upper arm 18 as shown in Figure 2. Motors 853 and 854 may be configured to move end effector coupler 22 to provide translational movement and rotation along in about three-dimensional axes. The computer platform 910 shown in Figure 9 can provide control input to the controller 846 that guides movement of the end effector coupler 22 to position a passive end effector, which is connected thereto, with a planned pose (i.e., location and angular orientation relative to defined 3D orthogonal reference axes) relative to an anatomical structure that is to be operated on during a planned surgical procedure. Motion control subsystem 840 may be configured to measure position of the end effector coupler 22 and/or the end effector 26 using integrated position sensors (e.g. encoders).

Figure 9 illustrates a block diagram of components of a surgical system that includes imaging devices (e.g., C-Arm, O-Arm, etc.) connected to a computer platform 910 which can be operationally connected to a camera tracking system component 6 (Fig. 3A) or 6' (Figs. 3B,3C) and/or to surgical robot 4 according to some embodiments of the present disclosure. Alternatively, at least some operations disclosed herein as being performed by the computer platform 910 may additionally or alternatively be performed by components of a surgical system.

Referring to Figure 9, the computer platform 910 includes a display 912, at least one processor circuit 914 (also referred to as a processor for brevity), at least one memory circuit 916 (also referred to as a memory for brevity) containing computer readable program code 918, and at least one network interface 902 (also referred to as a network interface for brevity). The display 912 may be part of an XR headset 920 in accordance with some embodiments of the present disclosure. The network interface 902 can be configured to connect to a C-Arm imaging device 104, an O-Arm imaging device 106, another medical imaging device, an image database 950 containing patient medical images, components of the surgical robot 4, and/or other electronic equipment.

When used with a surgical robot 4, the display 912 may correspond to the display 34 of Figure 2 and/or the tablet 890 of Figure 8 and/or the XR headset 920 that is operatively connected to the surgical robot 4, the network interface 902 may correspond to the platform network interface 812 of Figure 8, and the processor 914 may correspond to the computer 822 of Figure 8. The network interface 902 of the XR headset 920 may be configured to communicate through a wired network, e.g., thin wire ethernet, and/or through wireless RF transceiver link according to one or more wireless communication protocols, e.g., WLAN, 3GPP 4G and/or 5G (New Radio) cellular communication standards, etc.

The processor 914 may include one or more data processing circuits, such as a general purpose and/or special purpose processor, e.g., microprocessor and/or digital signal processor. The processor 914 is configured to execute the computer readable program code 918 in the memory 916 to perform operations, which may include some or all of the operations described herein as being performed for surgery planning, navigated surgery, and/or robotic surgery.

The processor 914 can operate to display on the display device 912 an image of a bone that is received from one of the imaging devices 104 and 106 and/or from the image database 950 through the network interface 902. The processor 914 receives an operator's definition of where an anatomical structure, i.e. one or more bones, shown in one or more images is to be cut, drilled, or have other surgical operation performed, such as by an operator touch selecting locations on the display 912 for planned surgical operation(s) or using a mouse-based cursor to define locations for planned operation(s).

The computer platform 910 can be configured to provide surgery planning functionality. The processor 914 can operate to display on the display device 912 and/or on the XR headset 920 an image of an anatomical structure, e.g., vertebra, that is received from one of the imaging devices 104 and 106 and/or from the image database 950 through the network interface 902. The processor 914 receives an operator's definition of where the anatomical structure shown in one or more images is to have a surgical procedure, e.g., screw placement, such as by the operator touch selecting locations on the display 912 for planned procedures or using a mouse-based cursor to define locations for planned procedures. When the image is displayed in the XR headset 920, the XR headset can be configured to sense in gesture-based commands formed by the wearer and/or sense voice based commands spoken by the wearer, which can be used to control selection among menu items and/or control how objects are displayed on the XR headset 920 as will be explained in further detail below.

The computer platform 910 can be configured to enable anatomy measurement, which can be particularly useful for spine surgery, like dimensional measurements of vertebrae and discs spacing.), etc. Some measurements can be automatic while some others involve human input or assistance. This computer platform 910 can allow an operator to choose the correct implant for a patient, including choice of size and alignment. As will be explained further below, a ML guidance system 1220 provides guidance to a user during pre-operative planning and during intra-operative surgical execution of the surgical plan. The ML guidance system enables automatic or semi-automatic (involving human input) selection of implant(s) and generation of the surgical plan.

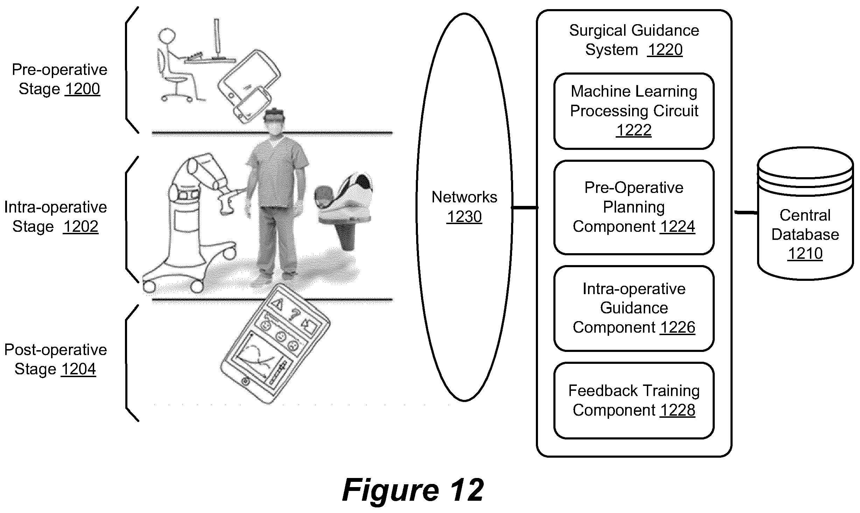

The surgical planning computer 910 enables automatic or semi-automatic segmentation (image processing) for CT images or other medical images. The surgical plan for a patient may be stored in a central database 1210 (Fig. 12) for retrieval by the surgical robot 800. During the surgery, the surgeon will choose which component of the surgical plan is being performed using a computer screen (e.g. touchscreen) or augmented reality interaction via, e.g., a head-mounted display. The surgical robot 4 may automatically move the end effector the robot arm to a planned pose so that a tool guided by the end effector is optimally oriented along a planned trajectory relative to the target location on the patient.

For a surgical plan involving inserting a spacer between vertebrae, for example, after creating space for the spacer, a surgeon may initiate guidance for a trajectory for the spacer insertion using a computer screen (e.g. touchscreen) or extended reality (XR) interaction (e.g., hand gesture based commands and/or voice based commands) via, e.g., the XR headset 920. The computer platform 910 can generate navigation information which provides visual guidance to the surgeon for performing the surgical procedure. When used with the surgical robot 4, the computer platform 910 can provide guidance that allows the surgical robot 4 to automatically or semi-automatically move the end effector 26 to a target pose so that the surgical tool for inserting the spacer is aligned with a planned trajectory toward the disc space ready to accept the spacer.

The system 900 may operate the XR headset 920 to display a three-dimensional (3D) computer generated representation of the spacer that can be viewed through the XR headset 920 overlaid on the target location on the spine. The computer generated representation is scaled and posed relative to the patient on the display screen under guidance of the computer platform 910, and the pose can be manipulated by a surgeon while viewed through the XR headset 920. A surgeon may, for example, manipulate the displayed computer-generated representation of the anatomical structure, the implant, a surgical tool, etc., using hand gesture based commands and/or voice based commands that are sensed by the XR headset 920.

For example, during the pre-operative stage a surgeon can view a displayed virtual handle on a virtual implant, and can manipulate (e.g., grab and move) the virtual handle to move the virtual implant to a desired pose and adjust a planned implant placement relative to a graphical representation of the patient's spine or other anatomical structure. Afterward, during surgery, the computer platform 910 could display navigation information through the XR headset 920 that facilitates the surgeon's ability to more accurately follow the surgical plan to insert the implant and/or to perform another surgical procedure on the spine. When the surgical procedure involves bone removal, the progress of bone removal, e.g., depth of cut, can be displayed in real-time through the XR headset 920.

The computer platform 910 may graphically illustrate one or more cutting planes intersecting the displayed anatomical structure at the locations selected by the operator for cutting the anatomical structure. The computer platform 910 also determines one or more sets of angular orientations and locations where the end effector coupler 22 must be positioned so a cutting plane of the surgical tool will be aligned with a target plane to perform the operator defined cuts, and stores the sets of angular orientations and locations as data in a surgical plan data structure. The computer platform 910 uses the known range of movement of the tool attachment mechanism of the passive end effector to determine where the end effector coupler 22 attached to the robot arm 20 needs to be positioned.

The computer subsystem 820 of the surgical robot 800 receives data from the surgical plan data structure and receives information from the camera tracking system component 6 indicating a present pose of an anatomical structure that is to be cut and indicating a present pose of the passive end effector and/or surgical saw tracked through DRAs. The computer subsystem 820 determines a pose of the target plane based on the surgical plan defining where the anatomical structure is to be cut and based on the pose of the anatomical structure, The computer subsystem 820 generates steering information based on comparison of the pose of the target plane and the pose of the surgical saw. The steering information indicates where the passive end effector needs to be moved so the cutting plane of the saw blade becomes aligned with the target plane and the saw blade becomes positioned a distance from the anatomical structure to be cut that is within the range of movement of the tool attachment mechanism of the passive end effector.

As explained above, a surgical robot includes a robot base, a robot arm connected to the robot base, and at least one motor operatively connected to move the robot arm relative to the robot base. The surgical robot also includes at least one controller, e.g. the computer subsystem 820 and the motion control subsystem 840, connected to the at least one motor and configured to perform operations.

An automated imaging system can be used in conjunction with the surgical planning computer 910 and/or the surgical system 2 to acquire pre-operative, intra-operative, post-operative, and/or real-time image data of a patient. In some embodiments, the automated imaging system is a C-arm imaging device or an O-arm^{®}. (O-arm^{®} is copyrighted by Medtronic Navigation, Inc. having a place of business in Louisville, Colo., USA) It may be desirable to take x-rays of a patient from a number of different positions, without the need for frequent manual repositioning of the patient which may be required in an x-ray system. C-arm x-ray diagnostic equipment may solve the problems of frequent manual repositioning and may be well known in the medical art of surgical and other interventional procedures. A C-arm includes an elongated C-shaped member terminating in opposing distal ends of the "C" shape. C-shaped member is attached to an x-ray source and an image receptor. The space within C-arm of the arm provides room for the physician to attend to the patient substantially free of interference from the x-ray support structure.

The C-arm is mounted to enable rotational movement of the arm in two degrees of freedom, (i.e. about two perpendicular axes in a spherical motion). C-arm is slidably mounted to an x-ray support structure, which allows orbiting rotational movement of the C-arm about its center of curvature, which may permit selective orientation of x-ray source and image receptor vertically and/or horizontally. The C-arm may also be laterally rotatable, (i.e. in a perpendicular direction relative to the orbiting direction to enable selectively adjustable positioning of x-ray source and image receptor relative to both the width and length of the patient). Spherically rotational aspects of the C-arm apparatus allow physicians to take x-rays of the patient at an optimal angle as determined with respect to the particular anatomical condition being imaged.

An O-arm^{®} includes a gantry housing which may enclose an image capturing portion, not illustrated. The image capturing portion includes an x-ray source and/or emission portion and an x-ray receiving and/or image receiving portion, which may be disposed about one hundred and eighty degrees from each other and mounted on a rotor relative to a track of the image capturing portion. The image capturing portion may be operable to rotate three hundred and sixty degrees during image acquisition. The image capturing portion may rotate around a central point and/or axis, allowing image data of the patient to be acquired from multiple directions or in multiple planes.

The O-arm^{®} with the gantry housing has a central opening for positioning around an object to be imaged, a source of radiation that is rotatable around the interior of gantry housing, which may be adapted to project radiation from a plurality of different projection angles. A detector system is adapted to detect the radiation at each projection angle to acquire object images from multiple projection planes in a quasi-simultaneous manner. The gantry may be attached to a support structure O-arm^{®} support structure, such as a wheeled mobile cart with wheels, in a cantilevered fashion. A positioning unit translates and/or tilts the gantry to a planned position and orientation, preferably under control of a computerized motion control system. The gantry may include a source and detector disposed opposite one another on the gantry. The source and detector may be secured to a motorized rotor, which may rotate the source and detector around the interior of the gantry in coordination with one another. The source may be pulsed at multiple positions and orientations over a partial and/or full three hundred and sixty degree rotation for multi-planar imaging of a targeted object located inside the gantry. The gantry may further comprise a rail and bearing system for guiding the rotor as it rotates, which may carry the source and detector. Both and/or either O-arm^{®} and C-arm may be used as automated imaging system to scan a patient and send information to the surgical system 2.

Images captured by the automated imaging system can be displayed a display device of the surgical planning computer 910, the surgical robot 800, and/or another component of the surgical system 2.

Figure 10 illustrates electrical components of the XR headset 920 that can be operatively connected to the computer platform 910, to one or more of the imaging devices, such as the C-arm imaging device 104, the O-arm imaging device 106, and/or the database 950, and/or to the surgical robot 800 in accordance with various embodiments of the present disclosure. The database 950 can be a repository for patient data, which can include historical medical images, medical history, etc.

The XR headset 920 provides an improved human interface for performing navigated surgical procedures. The XR headset 920 can be configured to provide functionalities, e.g., via the computer platform 910, that include without limitation any one or more of: identification of hand gesture based commands and/or voice based commands, display XR graphical objects on a display device 1050. The display device 1050 may a video projector, flat panel display, etc., which projects the displayed XR graphical objects on the display screen. The user can view the XR graphical objects as an overlay anchored to particular real-world objects viewed through the display screen. The XR headset 920 may additionally or alternatively be configured to display on the display screen 1050 video feeds from cameras mounted to one or more XR headsets 920 and other cameras.

Electrical components of the XR headset 920 can include a plurality of cameras 1040, a microphone 1042, a gesture sensor 1044, a pose sensor (e.g., inertial measurement unit (IMU)) 1046, a display module 1048 containing the display device 1050, and a wireless/wired communication interface 1052. The cameras 1040 of the XR headset may be visible light capturing cameras, near infrared capturing cameras, or a combination of both.

The cameras 1040 may be configured operate as the gesture sensor 1044 by capturing for identification user hand gestures performed within the field of view of the camera(s) 1040. Alternatively the gesture sensor 1044 may be a proximity sensor and/or a touch sensor that senses hand gestures performed proximately to the gesture sensor 1044 and/or senses physical contact, e.g. tapping on the sensor or the enclosure 1304. The pose sensor 1046, e.g., IMU, may include a multi-axis accelerometer, a tilt sensor, and/or another sensor that can sense rotation and/or acceleration of the XR headset 920 along one or more defined coordinate axes. Some or all of these electrical components may be contained in the component enclosure 1304 or may be contained in another enclosure configured to be worn elsewhere, such as on the hip or shoulder.

As explained above, the surgical system 2 includes a camera tracking system component 6/6' and a tracking subsystem 830 which may be part of the computer platform 910. The surgical system may include imaging devices (e.g., C-arm 104, O-arm 106, and/or image database 950) and/or a surgical robot 4. The tracking subsystem 830 is configured to determine a pose of DRAs attached to an anatomical structure, an end effector, a surgical tool, etc. A navigation controller 828 is configured to determine a target pose for the surgical tool relative to an anatomical structure based on a surgical plan, e.g., from a surgical planning function performed by the computer platform 910 of Fig. 9, defining where a surgical procedure is to be performed using the surgical tool on the anatomical structure and based on a pose of the anatomical structure determined by the tracking subsystem 830. The navigation controller 828 may be further configured to generate steering information based on the target pose for the surgical tool, the pose of the anatomical structure, and the pose of the surgical tool and/or the end effector, where the steering information indicates where the surgical tool and/or the end effector of a surgical robot should be moved to perform the surgical plan.

The electrical components of the XR headset 920 can be operatively connected to the electrical components of the computer platform 910 through a wired/wireless interface 1052. The electrical components of the XR headset 920 may be operatively connected, e.g., through the computer platform 910 or directly connected, to various imaging devices, e.g., the C-arm imaging device 104, the I/O-arm imaging device 106, the image database 950, and/or to other medical equipment through the wired/wireless interface 1052.

The surgical system 2 further includes at least one XR headset controller 1030 (also referred to as "XR headset controller" for brevity) that may reside in the XR headset 920, the computer platform 910, and/or in another system component connected via wired cables and/or wireless communication links. Various functionality is provided by software executed by the XR headset controller 1030. The XR headset controller 1030 is configured to receive navigation information from the navigation controller 828 which provides guidance to the user during the surgical procedure on an anatomical structure, and is configured to generate an XR image based on the navigation information for display on the display device 1050 for projection on the see-through display screen 1302.

The configuration of the display device 1050 relative to the display screen (also referred to as "see-through display screen ") 1302 is configured to display XR images in a manner such that when the user wearing the XR headset 920 looks through the display screen 1302 the XR images appear to be in the real world. The display screen 1302 can be positioned by the headband 1306 in front of the user's eyes.

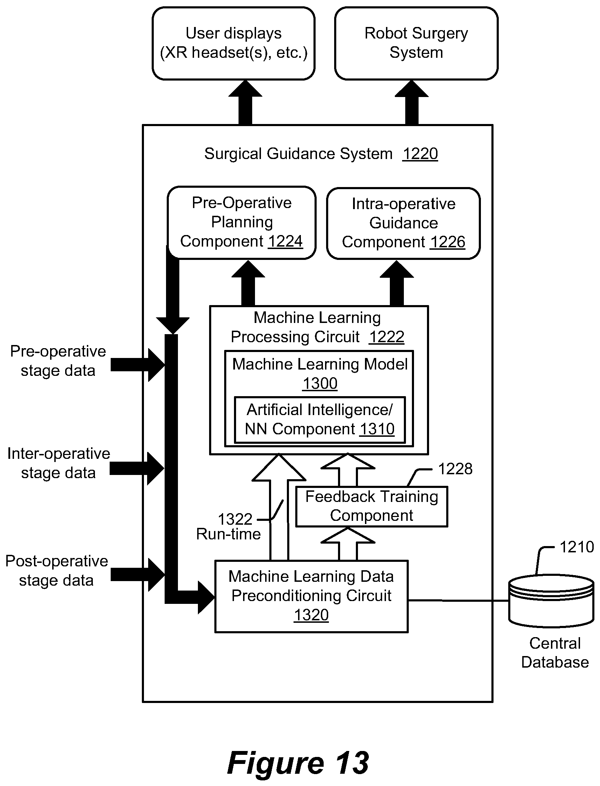

The XR headset controller 1030 can be within a housing that is configured to be worn on a user's head or elsewhere on the user's body while viewing the display screen 1302 or may be remotely located from the user viewing the display screen 1302 while being communicatively connected to the display screen 1302. The XR headset controller 1030 can be configured to operationally process signaling from the cameras 1040, the microphone 142, and/or the pose sensor 1046, and is connected to display XR images on the display device 1050 for user viewing on the display screen 1302. Thus, the XR headset controller 1030 illustrated as a circuit block within the XR headset 920 is to be understood as being operationally connected to other illustrated components of the XR headset 920 but not necessarily residing within a common housing (e.g., the electronic component enclosure 1304 of Fig. 13) or being otherwise transportable by the user. For example, the XR headset controller 1030 may reside within the computer platform 910 which, in turn, may reside within a housing of the computer tracking system component 6' shown in Figures 3B and 3C.

Figure 11 illustrates an example view through the display device 1050 of the XR headset 920 for providing navigation assistance to a user who is manipulating a surgical tool 1102 during a medical procedure in accordance with some embodiments of the present disclosure. Referring to Figure 11, when the surgical tool 1100 is brought in vicinity of a tracked anatomical structure (e.g., spinal vertebrae) so that dynamic reference arrays 1130 and 1132, connected to the surgical tool 1102, become within the field of view of the tracking cameras of the XR headsets 920 and/or the auxiliary tracking bar 46, a graphical representation 1100 of the tool can be displayed in 2D and/or 3D images in relation to a graphical representation 1110 of the anatomical structure (spine vertebrae). The user can use the viewed graphical representations to adjust a trajectory 1120 of the surgical tool 1102, which can be illustrated as extending from the graphical representation 1100 of the tool through the graphical representation 1610 of the anatomical structure. Alternatively, the trajectory 1120 may correspond to a planned trajectory for the tool to intercept the graphical representation 1110 of the anatomical structure, so that the user can align a trajectory of the graphical representation 1100 of the tool with the planned trajectory 1120. The XR headset 920 may also display textual information and other objects 1140. The dashed line 1150 extending across the viewed display screen represents an example division between different opacity level upper and lower bands of the XR headset 920.

Other types of XR images (virtual content) that can be displayed on the display device 1050 can include, but are not limited to any one or more of:
l) 2D Axial, Sagittal and/or Coronal views of patient anatomy;
2) overlay of planned vs currently tracked tool and surgical implant locations;
3) gallery of preoperative images;
4) video feeds from microscopes and other similar systems or remote video conferencing;
5) options and configuration settings and buttons;
6) floating 3D models of patient anatomy with surgical planning information;
7) real-time tracking of surgical instruments relative to floating patient anatomy;
8) augmented overlay of patient anatomy with instructions and guidance; and
9) augmented overlay of surgical equipment.

### MACHINE LEARNING SYSTEM FOR NAVIGATED ORTHOPEDIC SURGERIES:

As explained above, the degree of surgical outcome success is dependent on the surgeon's ability to design and implement the best surgical plan for a particular patient's needs. However, optimal design and implementation may not be achievable in view of inherent limitations in a surgeon's ability to adapt past practice experiences to a patient's particular spinal geometries and corrective needs when selecting among a myriad of combinations of medically accepted spinal surgery procedures and numerous available implants types and configurations. This suggests that there are problems that have not been addressed with previous medical procedures and related innovations.

There are potentially many variables that influence the outcome of the surgery:
- Planning: how to make adapt surgeries to be more patient-specific? How to consider current patient deformity from a model? What shall be target deformity correction?
- What implant type is the best for a selected patient? It is noted that there can be more than several dozens types of available implant types that a surgeon may be able to select among for a patient.

These variables result in a large number of possible combinations that a surgeon may need to select among for use in an orthopedic surgery for a selected patient.

The claimed invention is directed to a surgical guidance system that includes a machine learning processing circuit that processes data obtained and/or reported during pre-operative, intra-operative, and post-operative stages of surgery for patients. Over time, the machine learning processing circuit trains a machine learning model based on historical correlations and/or other trends determined between, for example, the variables (metrics or other data) that have been selected by surgeons during the pre-operative stage, the tracked movements during navigated surgery, and the resulting outcomes for patients. The training can include adapting rules of an artificial intelligence (AI) algorithm, rules of one or more sets of decision operations, and/or weights and/or firing thresholds of nodes of a neural network mode, to drive one or more defined key performance surgical outcomes toward one or more defined thresholds or other rule(s) being satisfied. The surgical guidance system processes pre-operative data for a new patent's characteristics through the machine learning model to provide navigated guidance to a surgeon during the pre-operative stage when generating a surgical plan with implant selection. The surgical plan can be provided to a navigation system to provide guidance to the surgeon during the intra-operative stage to assist the surgeon with execution of the surgical plan. Additionally, the surgical plan can be provided to a robot surgery system to control movements of a robot arm that assists the surgeon during execution of the surgical plan.

Although various embodiments are described in the context of machine learning whereby the machine learning model is trained over time, the machine learning model would typically not be adapted from a zero knowledge starting point. Instead, these and other embodiments may be used with a machine learning model that is pre-programmed based on human defined best practices for selecting among a set of defined surgical tools and implants to perform one or more defined surgical procedures on patients having defined characteristics.

Figure 12 illustrates a navigated surgery workflow which uses a surgical guidance system 1220 configured in accordance with some embodiments. Referring to Figure 12, three stages of workflow are illustrated: pre-operative stage 1200; intra-operative stage 1202; and post-operative stage 1204: During the pre-operative stage 1200, a user (e.g., surgeon) generates a surgical plan (case) based on analyzed patient images with assistance from the surgical guidance system 1220. During the intra-operative stage 1202, the user is provided navigated assistance by the surgical guidance system 1220 which may include operation of a surgical robot 4 for precise plan execution. During the post-operative stage 1204, post-operative feedback data characterizing surgery outcomes is collected by the surgical guidance system 1220, such as by patient measurements and/or patient surveys, etc. Data obtained across the three phases 1200, 1202, and 1204 can be stored in a central database 1210 for use by the surgical guidance system 1220 to train a machine learning model of a machine learning processing circuit 1222. In addition, other data not related one of the three phases 1200, 1202, and 1204 can be stored in a central database 1210 for use by the surgical guidance system 1220 to train a machine learning model of a machine learning processing circuit 1222.

The machine learning model can include artificial intelligence (AI) processes, neural network components, etc. The machine learning model is trained over time and used to generate surgical plans that result in improved surgical outcomes.

The example surgical guidance system 1220 shown in Figure 12 includes a preoperative planning component 1224, an intra-operative guidance component 1226, a machine learning processing circuit 1222, and a feedback training component 1228.

As will be explained in further detail below, the feedback training component 1228 is configured to obtain post-operative feedback data provided by distributed networked computers regarding surgical outcomes for a plurality of patients, and to train a machine learning model based on the post-operative feedback data. Although Figure 12 shows a single computer, e.g., smart phone, providing post-operative feedback data during the post-operative stage 1204 through one or more networks 1230 (e.g., public (Internet) networks and or private networks) to the surgical guidance system 1220 for storage in the central database 1210, it is to be understood that many network computers (e.g., hundreds of computers) would provide post-operative feedback data for each of many prior patients (e.g., hundreds of patients) to the surgical guidance system 1220 (i.e., to the feedback training component 1228) for use in training the machine learning model. Moreover, as explained in further detail below, the feedback training component 1228 can further train the machine learning model based on preoperative data (e.g., surgical plans) obtained during the pre-operative stage 1200 for the prior patients and based on intra-operative data obtained during the intra-operative stage 1202 (e.g., data indicating what surgical procedure(s) were used, what implant(s) were used, which tool(s) were used, 6 degree-of-freedom (DOF) tracked movements of the tool(s) during identified surgical procedure(s), etc.) for the prior patients. For example, the training can include adapting rules of an Al algorithm, rules of one or more sets of decision operations, and/or weights and/or firing thresholds of nodes of a neural network mode, to drive one or more outputs (e.g., surgical plan(s)) of the machine learning model 1300 toward one or more defined thresholds or other rule(s) being satisfied (e.g., defined key performance surgical outcomes indicated by the post-operative stage data).

A pre-operative planning component 1224 obtains pre-operative data from one of the distributed network computers characterizing a candidate patient who has a spinal condition considered for surgical correction, and generates a surgical plan for the candidate patient based on processing the pre-operative data through the machine learning model. The pre-operative planning component 1224 provides the surgical plan to a display device for review by a user, e.g., surgeon. Accordingly, the pre-operative planning component 1224 of the machine learning processing circuit 1222 generates a surgical plan for the candidate patient using the machine learning model which has been trained based on the post-operative feedback data regarding surgical outcomes for the prior patients. The training of the machine learning model can be repeated as more post-operative feedback is obtained by the feedback training component 1228 so that the surgical plans that are generated will result in continuing improvement of the resulting surgical outcomes for patients.

Figure 13 illustrates a block diagram of the surgical guidance system 1220 with associated data flows during the pre-operative, intra-operative, and post-operative stages, and shows surgical guidance being provided to user displays and to a robot surgery system.

Referring to Figure 13, the surgical guidance system 1220 includes the feedback training component 1228, the pre-operative planning component 1224, and the intra-operative guidance component 1226. The surgical guidance system 1220 also includes machine learning processing circuit 122 that includes machine learning module 1300, which may include an artificial intelligence component and/or neural network component 1310 as explained in further detail below. Although Figure 13 includes arrows on communication paths to show a primary direction of communication, it is to be understood that communication may occur in the opposite direction to the depicted arrows and can occur between elements for which arrows have not been shown to simplify the drawing.

### TRAINING THE MACHINE LEARNING MODEL:

The feedback training component 1228 can operate to obtain post-operative stage feedback data provided by distributed networked computers which characterizes surgical outcomes for prior patients who have completed rehabilitation therapy and/or surgery to attempt to remedy a spinal condition. The feedback training component 1228 may also operate obtain pre-operative stage data and/or inter-operative stage data for the prior patients. The pre-operative stage data can characterize the patients' and their spinal conditions. The inter-operative stage data can characterize the surgical procedure(s) planned for the prior patients, the surgical implant(s) planned for the prior patients (for spinal implant procedures), the surgical tool(s) planned for use on the prior patients, planned trajectories and movements of the tool(s) and/or surgical implant(s) during the surgical procedure(s), the amount of spinal decompression (for spinal decompression procedures), time duration measurements for various stages of the surgical procedures, levels of difficulty assessed with the various stages of the surgical procedures, and other information as discussed below. The feedback training component 1228 uses the obtained pre-operative stage data, inter-operative stage data, and post-operative stage data to train the machine learning model 1300.

The machine learning model 1300 can include an Al-powered algorithm component and/or neural network component that is trained to identify correlations between preoperative stage data, intra-operative stage data, and post-operative stage data as explained below.

In some embodiments, the feedback training component 1228 is configured to train the machine learning model 1300 based on the pre-operative stage data for each prior patient (also called "pre-operative feedback data"), where the data can include any one or more of: patient demographics (e.g., age, gender, BMI, race, comorbidities); patient medical history; and medical image analysis (e.g., spine curvature, vertebral body dimensions, vertebral endcap contours, foraminal area / volume, spinal canal area / volume, location of herniation and/or boney osteophytes, etc.).

The feedback training component 1228 can also be operative to train the machine learning model 1300 based on pre-operative stage data that characterizes for each prior patient details of the surgery that was planned for execution on that patient. Similarly, the feedback training component 1228 can also be configured to train the machine learning model 1300 based on intra-operative stage data (also called "intra-operative feedback data"), that is collected during the surgical procedures and characterizes for each prior patient details of the surgery that was performed on that patient. This pre-operative stage data and/or intra-operative stage data can include any one or more of:
(1) pre-operatively planned or intra-operatively used ("planned or used") procedure type
   (a) vertebral decompression,
   (b) vertebral diffusion, and
   (c) lumbar discectomy to remove herniated tissue and/or disc, insert spacer, etc.;
(2) planned or used type of implant(s) (e.g., vertebral bodies fusion spacer implant such as Globus Medical, Inc. lateral fixation spacer implant types of CALIBER-L expandable height spacer, TransContinental fixed height spacer, InterContinental spacer, PLYMOUTH spacer, ELSA expandable integrated lateral interbody fusion spacer, etc.);
(3) planned or used implant dimension sizing
   (a) length of implant,
   (b) width of implant,
   (c) height of implant (fixed anterior height, expandable anterior height range, posterior taper),
   (d) implant plate-spacer sagittal profile (e.g., 0°, 6°, 20°, 25° lordotic), and/or
   (e) implant screw length, diameter, insertion trajectory angle;
(4) planned or used volume of bone graft (e.g., Allograft, Bone Morphogenetic Protein (BMP), etc.) used with implant (e.g., added into and/or around implant);
(5) planned or used implant location placement and configuration relative to endplates;
   (a) implant insertion path and fixation location,
   (b) implant expansion amount,
   (c) amount of any additional autogenous bone graft insertion into graft access holes on the implant and surrounding disc space, and/or
   (d) trajectory and depth of prepared screw holes and tool(s) used (e.g., awl to perforate cortex, drill to create screw holes);
(6) planned or used types of tool(s) (e.g., implant insertion tool, awl, drill, disc box cutter, disc rongeurs, kerrisons, curettes, scrapers, and rasp, etc.) and may include planned or used trajectory relative to patient and 6DOF planned movements;
(7) planned or performed amount of spinal decompression;
(8) planned or used incision location on patient;
(9) planned or used cannula insertion path relative to patient;
(10) planned or used retractor configuration (e.g., retractor selection (e.g., Globus Minimal Access Retractor System (MARS)), blade length choice);
(11) planned or used retractor operation to obtain access to target location on spine (retractor positioning, blade insertion path and depth, blade lateral movement, blade angulation), etc.;
(12) planned or used amount of spinal disc preparation;
(13) planned or used amount of spinal disc preparation and created amount of disc space by removing portion of intervertebral disc and/or osteophytes, and which tool(s) were planned or used for the process(es) (e.g., disc box cutter, disc rongeurs, kerrisons, curettes, scrapers, and rasp);
(14) planned or used amount of endplate preparation, (e.g., amount of superficial layers of the cartilaginous endplates removed to expose bleeding bone), and which tool(s) were planned or used for the process(es) (e.g., scrapers and rasp);
(15) deviations between planned and used procedures;
(16) deviations between planned and used implant characteristics (e.g., deviation of an implant device size that is implanted into a patient during surgery from an implant device size defined by a surgical plan);
(17) deviations between planned and used implant positioning and/or insertion trajectory (e.g., data indicating deviation of implant device pose after implantation into a patient during surgery from an implant device pose defined by a pre-operative surgical plan);
(18) deviations between planned and intra-operatively achieved levels of spinal correction; and
(19) surgery events (e.g., problems, failures, errors, observations during the surgical procedure.

In some additional or alternative embodiments, the feedback training component 1228 is operative to train the machine learning model 1300 based on the post-operative stage data (also called "post-operative feedback data"), which may include any one or more of: patient reported outcome measures; measured outcomes (e.g., deformity correction measurements, Range of Motion (ROM) test, soft tissue balance measurements, kinematics measurements, curvature measurements, other functional outcomes); logged surgery events; and observation metrics. The logged surgery event can include timing, problems (e.g., deviation of robot axes positions from plan, deviation of end effector positions from plan, deviation of surgical tool positions from plan, deviation of implant device position from plan, deviation of implant fit from predicted, unplanned user repositioning of robot arm, deviation of action tool motion from plan, unplanned surgical steps, etc.), failures (e.g., surgeon prematurely stops use of surgical robot system before plan completion, etc.), and errors (e.g., deviation of predicted gap from actual gap; camera tracking system loss of tracking markers during procedure step, etc.). Some post-operative stage data may be collected using a mobile application (e.g., smartphone or other computer application) that can operate standalone or can be communicatively connected (e.g., WiFi or Bluetooth paired) with one or more patient wearable devices for systematic data collection (functional data and Patient-reported Outcome Measures PROMs) before and after spinal surgery.

The feedback training component 1228 may process the pre-operative stage data, intra-operative stage data, and/or post-operative stage data to form subsets of the data having similarities that satisfy a defined rule. Within each of the subsets, the feedback training component 1228 can identify correlations among at least some values of the data, and then train the machine learning model based on the correlations identified for each of the subsets. The training can operate to adapt rules of an Al algorithm, rules of one or more sets of decision operations, and/or weights and/or firing thresholds of nodes of a neural network based on the identified correlations to drive one or more outputs (e.g., surgical plan(s)) of the machine learning model 1300 toward one or more defined thresholds or other rule(s) being satisfied (e.g., defined key performance surgical outcomes indicated by the post-operative stage data).

More particularly, the training can cause the machine learning model 1300 to find similarities (a threshold level of correlation) among the sets of data obtained for a set of the previous patients and identify what has been learned to be the best surgical plan that has been known to be used for one or more prior surgical patients among that set of previous patients. Elements in the sets of data may have different weightings based on a defined or learned level of effect in the process to generate a surgical plan that will achieve the best surgical outcome for a patient.

In some embodiments, the machine learning model 1300 includes a neural network component including an input layer having input nodes, a sequence of hidden layers each having a plurality of combining nodes, and an output layer having output nodes. The machine learning model is processed by at least one processing circuit (i.e., of the machine learning processing circuit 1222) configured to provide different entries of the pre-operative data to different ones of the input nodes of the neural network model, and to generate a pre-operative surgical plan based on output of output nodes of the neural network component. The feedback training component 1228 may be configured to adapt weights and/or firing thresholds that are used by the combining nodes of the neural network component based on values of the preoperative stage data, intra-operative stage data, and/or post-operative stage data.

For example, during a run-time mode 1322 and/or a training mode using feedback training component 1228, the interconnected structure of the neural network between the input nodes of the input layer, the combining nodes of the hidden layers, and the output nodes of the output layer can cause the inputted (processed) data values to simultaneously be processed to influence the generated output values that are used to generate the surgical plan. Each of the input nodes in the input layer multiply the input data value by a weight that is assigned to the input node to generate a weighted node value. When the weighted node value exceeds a firing threshold assigned to the input node, the input node then provides the weighted node value to the combining nodes of a first one of the sequence of the hidden layers. The input node does not output the weighted node value unless if the condition is satisfied where the weighted node value exceeds the assigned firing threshold.

Furthermore, the neural network operates the combining nodes of the first one of the sequence of the hidden layers using weights that are assigned thereto to multiply and mathematically combine weighted node values provided by the input nodes to generate combined node values, and when the combined node value generated by one of the combining nodes exceeds a firing threshold assigned to the combining node to then provide the combined node value to the combining nodes of a next one of the sequence of the hidden layers. Furthermore, the neural network circuit operates the combining nodes of a last one of the sequence of hidden layers using weights that are assigned thereto to multiply and combine the combined node values provided by a plurality of combining nodes of a previous one of the sequence of hidden layers to generate combined node values, and when the combined node value generated by one of the combining nodes exceeds a firing threshold assigned to the combining node to then provide the combined node value to the output nodes of the output layer. Finally, the output nodes of the output layer is then operated to combine the combined node values from the last one of the sequences of hidden layers to generate the output values used for generating the surgical plan.

A machine learning data preconditioning circuit 1320 may be provided that preprocesses the obtained data, such as by providing normalization and/or weighting of the various types of obtained data, which is then provided to machine learning processing circuit 1222 during a run-time mode 1322 or to the feedback training component 1228 during a training mode for use in training the machine learning model 1300. In some embodiments the training is performed only during the training mode while in some other embodiments the training is performed continuously or at least occasionally during the run-time mode.

The pre-operative planning component 1224 contains pre-operative data from one of the distributed network computers characterizing a candidate (defined) patient, generates a surgical plan for the candidate patient based on processing the pre-operative data through the machine learning model 1300, and provides the surgical plan to a display device for review by a user, e.g., surgeon.

Thus, as explained above, the training can include adapting weights and/or firing thresholds of nodes of a neural network mode to drive one or more outputs (e.g., surgical plan(s)) of the machine learning model 1300 toward one or more defined thresholds or other rule(s) being satisfied (e.g., defined key performance surgical outcomes indicated by the post-operative stage data).

### USING MACHINE LEARNING MODEL FOR PRE-OPERATIVE PLANNING:

Various embodiments of the present disclosure can work with one or more of the above systems or with other existing or new systems to process pre-operative stage data obtained for a candidate patient for spinal corrective surgery, through the machine learning processing circuit 1222 to generate one or more ranked surgical plans.

The pre-operative stage data that is obtained for the candidate patient can include any one or more of: patient demographics (e.g., age, gender, BMI, race, comorbidities); patient medical history; and patient medical image analysis. The patient medical images can be obtained from 3D CT scans and/or other imaging technique(s) which enable topography measurements of vertebral endplate contours, disc spacing, vertebral body dimensions, spine curvature, foraminal area / volume, spinal canal area / volume, location of herniation and/or boney osteophytes, etc. The images may include anterior/posterior (A/P) and lateral images of the patient. The surgical guidance system 1220 or another system can operate to determine the topography measurements of vertebral endplate contours, disc spacing, vertebral body dimensions, spine curvature, etc.

Various embodiments herein may be used in combination with various presently available products, such as: GENU system from Globus Medical (provides pre-operative planning and intra-operative robot-assisted execution), MAKO Robotic System (Mako System) from Stryker (provides pre-operative planning and intra-operative robot-assisted execution); NAVIO (Navio System) from Smith and Nephew (provides intra-operative planning and execution); ROSA (Rosa System) from Zimmer Biomet (implements pre-operative planning, intra-operative execution assisted by a robot and post-operative follow-up using wearables and mobile application, e .g., mymobility).

The machine learning processing circuit 1222 can process the pre-operatively stage data obtained for the candidate patient through the machine learning model 1300 to generate a surgical plan or a ranked list of alternative surgical plans, where each of the alternative surgical plans has a ranking that is based on an estimated level of surgical outcome success that is predicted to be provided by the associated surgical plan.

The processing can operate to find similarity between new pre-operative stage data for a candidate patient who is being planned for surgery compared to what has been learned (trained into the machine learning model 1300) to be the best surgical plan that has been determined to have been used for one or more prior surgical patients having similar (a threshold level of correlation) of their pre-operative stage data to the new pre-operative stage data for the candidate patient.

The one or more surgical plans can be provided to the pre-operative planning component 1224 for display to a user, e.g., surgeon to enable the surgeon to review and accept components of the surgical plan or to modify components of the surgical plan. Modification of a surgical plan may trigger the machine learning processing circuit 1222 to repeat the processing on the modified surgical plan to provide a revised estimate of the level of surgical outcome success that is predicted to be provided by the modified surgical plan. The estimate of the level of surgical outcome success that is predicted to be provided by the modified surgical plan can be based on identifying similarities what has been learned through training from the data collected in the central database 1210 between the historical inputs (pre-operative and/or intra-operative stage data) and the historical surgical outcomes (post-operative stage data).

These embodiments may be used for pre-operative planning (i.e., with or without an artificial intelligence (AI) based, rule based, or neural network based planning assistant) with a dashboard provided through which the user can review previous patient performances and summary statistics of other measures present in the central database 1210.

This process enables the user to understand during the pre-operative planning stage how the components of the surgical plan affect the estimated level of surgical outcome success, and can result in the surgeon producing a surgical plan that is customized for the unique characteristics of the candidate patient and has the highest likelihood of resulting in an optimal surgical outcome. Moreover, the user can adjust components of the surgical plan and immediately observe the effect of such adjustment on the surgical outcome.

Each surgical plan that is generated may characterize any one or more of:
(1) procedure type
(2) type of implant(s) (e.g., vertebral bodies fusion spacer implant such as Globus Medical, Inc. lateral fixation spacer implant types of CALIBER-L expandable height spacer, TransContinental fixed height spacer, InterContinental spacer, PLYMOUTH spacer, ELSA expandable integrated lateral interbody fusion spacer, etc.);
(3) implant dimension sizing
   (a) length of implant,
   (b) width of implant,
   (c) height of implant (fixed anterior height, expandable anterior height range, posterior taper),
   (d) implant plate-spacer sagittal profile (e.g., 0°, 6°, 20°, 25° lordotic), and/or
   (e) implant screw length, diameter, insertion trajectory angle;
(4) volume of bone graft (e.g., Allograft, Bone Morphogenetic Protein (BMP), etc.) used with implant (e.g., added into and/or around implant);
(5) implant location placement and configuration relative to endplates;
   (a) implant insertion path and fixation location,
   (b) implant expansion amount,
   (c) amount of any additional autogenous bone graft insertion into graft access holes on the implant and surrounding disc space, and/or
   (d) trajectory and depth of prepared screw holes and tool(s) used (e.g., awl to perforate cortex, drill to create screw holes);
(6) planned or used types of tool(s) (e.g., implant insertion tool, awl, drill, disc box cutter, disc rongeurs, kerrisons, curettes, scrapers, and rasp, etc.) and may include planned or used trajectory relative to patient and 6DOF planned movements;
(7) amount of spinal decompression;
(8) incision location on patient;
(9) cannula insertion path relative to patient;
(10) retractor configuration (e.g., retractor selection (e.g., Globus Minimal Access Retractor System (MARS)), blade length choice);
(11) retractor operation to obtain access to target location on spine (retractor positioning, blade insertion path and depth, blade lateral movement, blade angulation), etc.;
(12) process for creating disc space through planned removal of portion of intervertebral disc and/or osteophytes, and which tool(s) are planned for use during the process(es) (e.g., disc box cutter, disc rongeurs, kerrisons, curettes, scrapers, and rasp);
(13) process for endplate preparation, (e.g., amount of superficial layers of the cartilaginous endplates to be removed to expose bleeding bone), and which tool(s) are planned for the process(es) (e.g., scrapers and rasp);
(14) estimated level of surgical outcome success that is predicted to be provided by the associated surgical plan
   (a) estimated post-surgery deformity correction (e.g., estimated spinal curvature, disc spacing),
   (b) estimated post-surgery implant fit;
   (c) estimated patient reported outcome measures,
   (d) estimated post-surgery Range of Motion (ROM),
   (e) other estimated post-surgery functional outcomes,
   (f) duration of planned surgical procedure phases,
   (g) identification of deviations between planned and used procedures;
   (h) deviations between planned and used implant characteristics (e.g., deviation of an implant device size that is implanted into a patient during surgery from an implant device size defined by a surgical plan);
   (i) deviations between planned and used implant positioning and/or insertion trajectory (e.g., data indicating deviation of implant device pose after implantation into a patient during surgery from an implant device pose defined by a pre-operative surgical plan);
   (j) deviations between planned and intra-operatively achieved levels of spinal correction; and
   (k) probability of occurrence of defined surgery problematic events
      (i) camera tracking system loss of tracking markers during procedure step,
      (ii) deviation of planned implant device type and/or dimensions from surgical implant,
      (iii) deviation of planned implant device position from post-surgery implant fixation position,
      (iv) deviation of planned implant device fit from post-surgery implant fit,
      (v) deviation of predicted disk spacing gap from post-surgery gap,
      (vi) during navigated surgery need for user to cause deviation of surgical tool positions from plan,
      (vii) during robot assisted surgery need for user to cause deviation of robot axes positions from plan, and
      (viii) during robot assisted surgery need for user to cause deviation of end effector positions from plan.

Figures 14 and 15 illustrate a planned surgical procedure which includes using a selected surgical tool 1400 which is adapted for insertion of a selected sizing of CALIBER-L expandable height spacer 1500 into a planned pose within a disc gap 1510 that will be created between adjacent vertebrae 1410a and 1410b of a spine 1420. During pre-operative planning, the pre-operative planning component 1224 can display the surgical procedure to the user using graphical model representations of the surgical tool 1400, the spacer 1500 and the spine 1420. The spine 1420 can be represented by a graphical model which may be adapted based on data obtained from patient medical image(s) or may be represented by the patient medical image(s) themselves. During the intra-operative stage for execution of the surgical plan, the intra-operative guidance component 1226 can display navigation information that is viewed as being overlaid on the patient, and which can be posed to provide trajectory navigation into the patient's spine.

The surgical plan output by the machine learning processing circuit 1222 process the pre-operative stage data for a candidate patient to provide the one or more candidate surgical plans and the computed estimated level of surgical outcome success. In the next steps, the user can modify the plan, e.g., the implant size and placement, and observe results of such modifications by the surgical guidance system 1220 providing updated visual feedback displayed on the medical images. When the user indicates acceptability of the plan, the plan becomes validated as approved for surgery.

The surgical plan generated by the surgical guidance system 1220 via the machine learning processing circuit 1222 can be displayed to the user (e.g., surgeon) to help a surgeon track performance over time using a visual dashboard, and/or enable the surgeon to improve performance by reviewing easily accessible analysis data from, e.g., the surgeon's own surgeries and/or surgeries performed by other surgeons (which may be anonymized).

The pre-operative planning component 1224 may provide data indicating components of the surgical plan to the navigation controller 828 and computer platform 910 (Fig. 10) which generates graphical representations of the surgical plan for display on user display(s), such as the display device within the XR headset 920 (Fig. 9) as an overlay on the patient medical images and/or combined with computer generated models based on the patient medical images, to provide navigated guidance to the users while executing the planned surgical procedure.

The intra-operative guidance component 1226 may provide data indicating components of the surgical plan to the robot surgery system to provide automated or semiautomated navigated performance of the planned surgical procedure. For example, the intra-operative guidance component 1226 may provide data indicating a sequence of poses of the end effector to at least one controller of the surgical robot 4 (Fig. 9) to control a sequence of movements of the end effector attached to an arm of the surgical robot so a surgical becomes sequentially aligned with the poses relative to the patient.

### CORRESPONDING OPERATIONAL PROCESSES:

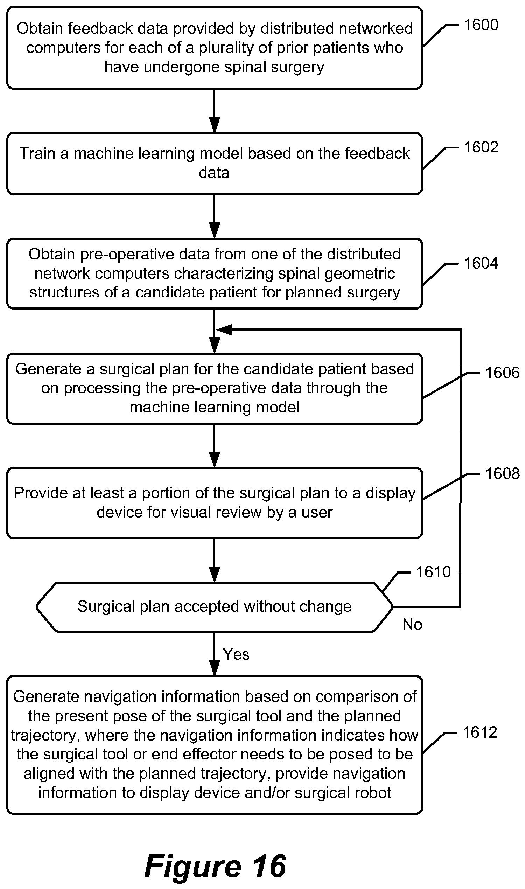

Figure 16 illustrates a flowchart of operations that can be performed by a surgical system which may include the surgical guidance system 1220 (Fig. 13), the computer platform 910 (Fig. 10), the camera tracking system component 6/6' (Fig. 10), and the surgical robot 4 (Fig. 10).

Referring to Figure 16, the surgical system includes a surgical guidance system for computer assisted navigation during surgery. The surgical guidance system is operative to obtain 1600 feedback data provided by distributed networked computers for each of a plurality of prior patients who have undergone spinal surgery. The feedback data characterizes spinal geometric structures of the prior patient, characterizing a surgical procedure performed on the prior patient, characterizing an implant device that was surgically implanted into the prior patient's spine, and characterizing the prior patient's surgical outcome. The surgical guidance system is further operative to train 1602 a machine learning model based on the feedback data. The surgical guidance system is further operative to obtain 1604 pre-operative data from one of the distributed network computers characterizing spinal geometric structures of a candidate patient for planned surgery, and generate 1606 a surgical plan for the candidate patient based on processing the pre-operative data through the machine learning model. The surgical plan identifies type and dimension sizing of a spinal implant device for surgical implantation in the spine of the candidate patient and identifies a planned trajectory for implantation of the spinal implant device. At least a portion surgical plan is provided 1608 to a display device for visual review by user.

As explained above, the pre-operative planning component 1224 (Fig. 13) can display the surgical plan to a user, e.g., surgeon, to enable the surgeon to review and accept components of the surgical plan or modify components of the surgical plan. Modification of a surgical plan may trigger the machine learning processing circuit 1222 (Fig. 13) to repeat the processing on the modified surgical plan to provide a revised estimate of the level of surgical outcome success that is predicted to be provided by the modified surgical plan. Once the surgical plan is determined 1610 to be accepted without further change (i.e., finalized surgical plan), the surgical plan can be processed to generate navigation information.

At least one controller (e.g., navigation controller 828) can operative to generate navigation information based on comparison of the present pose of a surgical tool and/or a robot end effector and the planned trajectory for movement of the surgical tool, and can provide the navigation information to display device (e.g., XR headset 920). The navigation information can indicate how the surgical tool or an end effector of a surgical robot needs to be posed to be aligned with the planned trajectory.

In some embodiments, the navigation information is provided to a surgical robot which includes a robot base, a robot arm connected to the robot base, and at least one motor operatively connected to move the robot arm relative to the robot base. The robot arm is configured to connect to an end effector which guides movement of the surgical tool. The at least one controller is connected to the at least one motor and operative to determine a pose of the end effector relative to a planned pose of the end effector while guiding movement of the surgical tool along the planned trajectory during implantation of the spinal implant device, and generate navigation information based on comparison of the planned pose and the determined pose of the end effector, wherein the navigation information indicates where the end effector needs to be moved to become aligned with the planned pose so the surgical tool will be guided by the end effector along the planned trajectory toward the patient.

In some embodiments, the at least one controller is operative to autonomously or semi-autonomously control movement of the at least one motor based on the navigation information to reposition the end effector so the determined pose of the end-effector becomes aligned with the planned pose. In some other embodiments, the at least one controller is operative to provide the navigation information to a display device (e.g., acts are headset 920) for display to visually guide an operators movement of the end effector so the determined pose of the end effector becomes aligned with the planned pose.

As explained above, the machine learning model may be operative to process the pre-operative data to output the surgical plan identifying type and dimension sizing of a spinal implant device proposed for surgical implantation in the spine of the candidate patient.

The machine learning model may be further operative to process the pre-operative data to output the surgical plan with further identification of an estimated level of surgical outcome success predicted for the candidate patient from surgical implantation of the spinal implant device in the spine of the candidate patient, where the estimated level of surgical outcome success indicates a most likely patient reported outcome measure or spinal deformity correction measurement that will be obtained by surgical implantation of the spinal implant device in the spine of the candidate patient.

The machine learning model may be further operative to process the pre-operative data to output the surgical plan with further identification of a planned pose for implantation of the spinal implant device in the spine of the candidate patient.

The surgical guidance system may operate to determine a planned trajectory for implantation of the spinal implant device to the planned pose in the spine of the candidate patient identified by the surgical plan, obtain from a camera tracking system a present pose of a surgical tool being used to implant the spinal implant device in the spine of the candidate patient, generate navigation information based on comparison of the present pose of the surgical tool and the planned trajectory, wherein the navigation information indicates how the surgical tool needs to be posed to be aligned with the planned trajectory, and provide at least first part of the navigation information to a display device. The surgical guidance system may be further operative to provide at least second part of the navigation information to a surgical robot to control movement of a robot arm having an end effector which guides movement of the surgical tool. The at least second part of the navigation information indicates where the end effector needs to be moved so the surgical tool will be guided by the end effector along the planned trajectory for implantation of the spinal implant device to the planned pose of the spinal implant device in the spine of the candidate patient.

The machine learning model may be further operative to process the pre-operative data to output the surgical plan with further identification of a portion of the patient's spinal disc that is to be removed to allow insertion of a spacer type of the spinal implant device.

The surgical guidance system may be operative to process the surgical plan to obtain a three-dimensional model of the spinal implant device and provide a graphical representation of the three-dimensional model for display though the display device within an extended reality (XR) headset as an overlay on the candidate patient.

The feedback data used to train the machine learning model and which characterizes the prior patient's surgical outcome, may include post-operative feedback data characterizing a patient reported outcome measure or a spinal deformity correction measurement. The feedback data used to train the machine learning model may characterize a spinal surgery procedure type performed on the prior patient and a type and dimension sizing of a spinal implant device that was implanted in the prior patient. The feedback data used to train the machine learning model may characterize a volume of bone graft used with the spinal implant device when implanted in the prior patient.

The feedback data used to train the machine learning model may characterize at least one of: deviation between a planned level of spinal correction for the prior patient planned during a pre-operative stage and an achieved level of spinal correction for the prior patient measured during an intra-operative stage or post-operative stage; deviation between a planned surgical procedure that was planned during a pre-operative stage and a used surgical procedure that was performed on the prior patient during an intra-operative stage; deviation between a type and dimension sizing of a spinal implant device that was planned during a pre-operative stage and a used type and dimension sizing of a spinal implant device that was implanted into the prior patient during an intra-operative stage; deviation between a planned pose of a spinal implant device for fixation into the spine of the prior patient planned during a pre-operative stage and a used pose of the spinal implant device following fixation into the spine of the prior patient during an intra-operative stage; and deviation between a planned insertion trajectory for implantation of the spinal implant device into the spine of the prior patient planned during a pre-operative stage and a used trajectory that the spinal implant device was moved along when implanted into the spine of the prior patient during an intra-operative stage.

The surgical guidance system may be further operative to: form subsets of the feedback data having similarities that satisfy a defined rule; within each of the subsets, identify correlations among at least some values of the feedback data; and train the machine learning model based on the correlations identified for each of the subsets.

The machine learning model may include: a neural network component including an input layer having input nodes, a sequence of hidden layers each having a plurality of combining nodes, and an output layer having output nodes; and at least one processing circuit operative to provide different entries of the pre-operative data to different ones of the input nodes of the neural network model, and to generate the surgical plan based on output of output nodes of the neural network component. The feedback training component may operative to adapt weights and/or firing thresholds that are used by the combining nodes of the neural network component based on values of the feedback data.

### Further Definitions and Embodiments:

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included in the present invention, as long as they fall within the scope of the appended claims. Accordingly, the above disclosed subject matter is to be considered illustrative, and not restrictive, and the appended examples of embodiments are intended to cover all such modifications, enhancements, and other embodiments, which fall within the scope of the present claims. Thus, to the maximum extent allowed by law, the scope of present inventive concepts are to be determined by the broadest permissible interpretation of the appended claims, and shall not be restricted or limited by the foregoing detailed description.

## Claims

1. A surgical system comprising:
- a surgical guidance system (1220) for computer assisted navigation during spinal surgery, the surgical guidance system operative to:
- obtain feedback data from one or more distributed networked computers (1230) associated with each of a plurality of prior patients who have undergone spinal surgery, the feedback data characterizing spinal geometric structures of the prior patient, characterizing a surgical procedure performed on the prior patient (1200), characterizing an implant device that was surgically implanted into the prior patient's spine, and characterizing the prior patient's surgical outcome (1204);
- train a machine learning model (1300) based on the feedback data;
- obtain pre-operative data (1200) from one of the distributed network computers characterizing spinal geometric structures of a candidate patient for planned surgery;
- generate a surgical plan for the candidate patient based on processing the pre-operative data through the machine learning model (1300); and
- provide at least a portion of the surgical plan to a display device for visual review by a user, wherein the surgical plan identifies type and dimension sizing of a spinal implant device for surgical implantation in the spine of the candidate patient and identifies a planned trajectory (1120) for implantation of the spinal implant device;
- a tracking system (6; 6') operative to determine a present pose of a surgical tool (1102) being used to implant the spinal implant device in the spine of the candidate patient; and
- at least one controller (828, 846) operative to generate navigation information based on comparison of the present pose of the surgical tool and the planned trajectory, wherein the navigation information indicates how the surgical tool needs to be posed to be aligned with the planned trajectory, and provide the navigation information to a display device (34),
the surgical system further comprising:
- a surgical robot (14) including
- a robot base (10),
- a robot arm connected to the robot base, the robot arm configured to connect to an end effector (100) which guides movement of the surgical tool (1102), and
- at least one motor operatively connected to move the robot arm (18,20) relative to the robot base,
- wherein the at least one controller is connected to the at least one motor and operative to
- determine a pose of the end effector relative to a planned pose of the end effector while guiding movement of the surgical tool along the planned trajectory during implantation of the spinal implant device, and
- generate navigation information based on comparison of the planned pose and the determined pose of the end effector, wherein the navigation information indicates where the end effector needs to be moved to become aligned with the planned pose so the surgical tool will be guided by the end effector along the planned trajectory toward the patient,
- an extended reality, XR, headset (920) including the display device, the at least one controller being operative to generate a graphical representation of the navigation information that is provided to the display device of the XR headset to guide operator movement of the surgical tool along the planned trajectory,
- dynamic reference arrays (52), DRAs, which are attached or formed on the XR headset, the end effector, the surgical robot, the surgical tool and an anatomical structure of a patient,
- a 3D localization system and a computer platform (910) which is configured to receive 3D or 2D image scan, the computer platform in combination with the 3D localization system being configured to track in real-time the pose of the DRA so as to determine the pose of the DRA in any multidimensional space in relation to the anatomical structure of the patient.

2. The surgical system of Claim 1, wherein the machine learning model is operative to: process the pre-operative data to output the surgical plan identifying type and dimension sizing of a spinal implant device proposed for surgical implantation in the spine of the candidate patient.

3. The surgical system of Claim 2, wherein the machine learning model (1300) is further operative to process the pre-operative data to output the surgical plan with further identification of an estimated level of surgical outcome success predicted for the candidate patient from surgical implantation of the spinal implant device in the spine of the candidate patient, wherein the estimated level of surgical outcome success indicates a most likely patient reported outcome measure or spinal deformity correction measurement that will be obtained by surgical implantation of the spinal implant device in the spine of the candidate patient.

4. The surgical system of Claim 2, wherein the machine learning model (1300) is further operative to process the pre-operative data to output the surgical plan with further identification of a planned pose for implantation of the spinal implant device in the spine of the candidate patient.

5. The surgical system of Claim 4, further operative to:
- determine a planned trajectory (1120) for implantation of the spinal implant device to the planned pose in the spine of the candidate patient identified by the surgical plan;
- obtain from a camera tracking system a present pose of a surgical tool being used to implant the spinal implant device in the spine of the candidate patient;
- generate navigation information based on comparison of the present pose of the surgical tool (1102) and the planned trajectory, wherein the navigation information indicates how the surgical tool needs to be posed to be aligned with the planned trajectory (1120); and
- provide at least first part of the navigation information to a display device.

6. The surgical system of Claim 5, further operative to: provide at least second part of the navigation information to the surgical robot (14) to control movement of a robot arm having an end effector which guides movement of the surgical tool, wherein the at least second part of the navigation information indicates where the end effector needs to be moved so the surgical tool will be guided by the end effector along the planned trajectory for implantation of the spinal implant device to the planned pose of the spinal implant device in the spine of the candidate patient.

7. The surgical system of Claim 1, wherein the feedback data used to train the machine learning model (1300) and which characterizes the prior patient's surgical outcome, includes post-operative feedback data characterizing a patient reported outcome measure or a spinal deformity correction measurement.

8. The surgical system of Claim 1, wherein the feedback data used to train the machine learning model (1300) characterizes a spinal surgery procedure type performed on the prior patient and a type and dimension sizing of a spinal implant device that was implanted in the prior patient.

9. The surgical system of Claim 8, wherein the feedback data used to train the machine learning model (1300) further characterizes a volume of bone graft used with the spinal implant device when implanted in the prior patient, and wherein preferably the feedback data used to train the machine learning model further characterizes at least one of:
- deviation between a planned level of spinal correction for the prior patient planned during a pre-operative stage and an achieved level of spinal correction for the prior patient measured during an intra-operative stage or post-operative stage;
- deviation between a planned surgical procedure that was planned during a pre-operative stage and a used surgical procedure that was performed on the prior patient during an intra-operative stage;
- deviation between a type and dimension sizing of a spinal implant device that was planned during a pre-operative stage and a used type and dimension sizing of a spinal implant device that was implanted into the prior patient during an intra-operative stage;
- deviation between a planned pose of a spinal implant device for fixation into the spine of the prior patient planned during a pre-operative stage and a used pose of the spinal implant device following fixation into the spine of the prior patient during an intra-operative stage; and
- deviation between a planned insertion trajectory (1120) for implantation of the spinal implant device into the spine of the prior patient planned during a pre-operative stage and a used trajectory that the spinal implant device was moved along when implanted into the spine of the prior patient during an intra-operative stage.

10. The surgical system of Claim 1, wherein the machine learning model comprises:
- a neural network component including an input layer having input nodes, a sequence of hidden layers each having a plurality of combining nodes, and an output layer having output nodes; and
- at least one processing circuit operative to provide different entries of the pre-operative data to different ones of the input nodes of the neural network model, and to generate the surgical plan based on output of output nodes of the neural network component and preferably further comprising a feedback training component operative to: adapt weights and/or firing thresholds that are used by the combining nodes of the neural network component based on values of the feedback data.

11. The surgical system according to any of preceding claims, wherein the extended reality includes a plurality of cameras (1040), a microphone (1042), a gesture sensor (1044), a pose sensor (1046), a display module (1048) containing the display device (1050), and a wireless or wired communication interface (1052), the cameras (1040) being configured operate as the gesture sensor (1044) by capturing for identification user hand gestures performed within the field of view of the camera(s) (1040).

## Patentansprüche

1. Chirurgisches System, umfassend:
- ein chirurgisches Führungssystem (1220) zur computergestützten Navigation während der Wirbelsäulenchirurgie, wobei das chirurgische Führungssystem betreibbar ist, um:
- Rückmeldungsdaten von einem oder mehreren verteilten vernetzten Computern (1230) zu erhalten, die jedem einer Vielzahl von früheren Patienten zugeordnet sind, die einer Wirbelsäulenchirurgie unterzogen wurden, wobei die Rückmeldungsdaten die geometrischen Wirbelsäulenstrukturen des früheren Patienten charakterisieren, ein chirurgisches Verfahren charakterisieren, das an dem früheren Patienten (1200) durchgeführt wird, eine Implantatvorrichtung charakterisieren, die chirurgisch in die Wirbelsäule des früheren Patienten implantiert wurde, und das chirurgische Ergebnis des früheren Patienten (1204) charakterisieren;
- ein Maschinenlernmodell (1300) basierend auf den Rückmeldungsdaten zu trainieren;
- präoperative Daten (1200) von einem der verteilten Netzwerkcomputer zu erhalten, die die geometrischen Wirbelsäulenstrukturen eines Kandidatenpatienten für die geplante Chirurgie charakterisieren;
- einen chirurgischen Plan für den Kandidatenpatienten basierend auf der Verarbeitung der präoperativen Daten durch das Maschinenlernmodell (1300) zu erzeugen; und
- mindestens einen Teil des chirurgischen Plans für eine Anzeigevorrichtung zur visuellen Überprüfung durch einen Benutzer bereitzustellen, wobei der chirurgische Plan die Typ- und Dimensionsgröße einer Wirbelsäulenimplantatvorrichtung identifiziert; - eine geplante Trajektorie (1120) für die Implantation der Wirbelsäulenimplantatvorrichtung zu identifizieren;
- ein Verfolgungssystem (6; 6'), das betreibbar ist, um eine aktuelle Pose eines chirurgischen Werkzeugs (1102) zu bestimmen, das verwendet wird, um die Wirbelsäulenimplantatvorrichtung in die Wirbelsäule des Kandidatenpatienten zu implantieren;
und
- mindestens eine Steuerung (828, 846), die betreibbar ist, um Navigationsinformationen basierend auf einem Vergleich der aktuellen Pose des chirurgischen Werkzeugs und der geplanten Trajektorie zu erzeugen, wobei die Navigationsinformationen angeben, wie das chirurgische Werkzeug posiert werden muss, um mit der geplanten Trajektorie ausgerichtet zu werden, und die Navigationsinformationen für eine Anzeigevorrichtung (34) bereitzustellen,
wobei das chirurgische System ferner umfasst:
- einen chirurgischen Roboter (14), umfassend
- eine Roboterbasis (10),
- einen Roboterarm, der mit der Roboterbasis verbunden ist, wobei der Roboterarm konfiguriert ist, um mit einem Endeffektor (100) verbunden zu werden, der die Bewegung des chirurgischen Werkzeugs (1102) führt, und
- mindestens einen Motor, der betriebsfähig verbunden ist, um den Roboterarm (18, 20) relativ zu der Roboterbasis zu bewegen,
wobei die mindestens eine Steuerung mit dem mindestens einen Motor verbunden ist und betreibbar ist, um
- eine Pose des Endeffektors relativ zu einer geplanten Pose des Endeffektors zu bestimmen, während die Bewegung des chirurgischen Werkzeugs entlang der geplanten Trajektorie während der Implantation der Wirbelsäulenimplantatvorrichtung geführt wird, und
- Navigationsinformationen basierend auf einem Vergleich der geplanten Pose und der bestimmten Pose des Endeffektors zu erzeugen, wobei die Navigationsinformationen angeben, wo der Endeffektor bewegt werden muss, um mit der geplanten Pose ausgerichtet zu werden, so dass das chirurgische Werkzeug durch den Endeffektor entlang der geplanten Trajektorie zu dem Patienten geführt wird,
- ein erweitertes Realitäts-, XR-, Headset (920), das die Anzeigevorrichtung umfasst, wobei die mindestens eine Steuerung betreibbar ist, um eine grafische Darstellung der Navigationsinformationen zu erzeugen, die für die Anzeigevorrichtung des XR-Headsets bereitgestellt wird, um die Bedienerbewegung des chirurgischen Werkzeugs entlang der geplanten Trajektorie zu führen,
- dynamische Referenzarrays (52), DRAs, die an dem XR-Headset, dem Endeffektor, dem chirurgischen Roboter, dem chirurgischen Werkzeug und einer anatomischen Struktur eines Patienten angebracht oder ausgebildet sind,
- ein 3D-Lokalisierungssystem und eine Computerplattform (910), die konfiguriert ist, um einen 3D- oder 2D-Bildscan zu empfangen, wobei die Computerplattform in Kombination mit dem 3D-Lokalisierungssystem konfiguriert ist, um die Pose des DRA in Echtzeit zu verfolgen, um die Pose des DRA in einem beliebigen mehrdimensionalen Raum in Bezug auf die anatomische Struktur des Patienten zu bestimmen.

2. Chirurgisches System nach Anspruch 1, wobei das Maschinenlernmodell betreibbar ist, um: die präoperativen Daten zu verarbeiten, um den chirurgischen Plan auszugeben, der die Typ- und Dimensionsgröße einer Wirbelsäulenimplantatvorrichtung identifiziert, die für die chirurgische Implantation in die Wirbelsäule des Kandidatenpatienten vorgeschlagen wird.

3. Chirurgisches System nach Anspruch 2, wobei das Maschinenlernmodell (1300) ferner betreibbar ist, um die präoperativen Daten zu verarbeiten, um den chirurgischen Plan mit weiterer Identifikation eines geschätzten Niveaus des Erfolgs des chirurgischen Ergebnisses auszugeben, das für den Kandidatenpatienten aus der chirurgischen Implantation der Wirbelsäulenimplantatvorrichtung in die Wirbelsäule des Kandidatenpatienten vorhergesagt wird, wobei das geschätzte Niveau des Erfolgs des chirurgischen Ergebnisses eine wahrscheinlichste vom Patienten gemeldete Ergebnismessung oder Korrekturmessung der Wirbelsäulenverformung anzeigt, die durch die chirurgische Implantation der Wirbelsäulenimplantatvorrichtung in die Wirbelsäule des Kandidatenpatienten erhalten wird.

4. Chirurgisches System nach Anspruch 2, wobei das Maschinenlernmodell (1300) ferner betreibbar ist, um die präoperativen Daten zu verarbeiten, um den chirurgischen Plan mit weiterer Identifikation einer geplanten Pose für die Implantation der Wirbelsäulenimplantatvorrichtung in die Wirbelsäule des Kandidatenpatienten auszugeben.

5. Chirurgisches System nach Anspruch 4, das ferner betreibbar ist, um:
- eine geplante Trajektorie (1120) für die Implantation der Wirbelsäulenimplantatvorrichtung zu der geplanten Pose in der Wirbelsäule des Kandidatenpatienten zu bestimmen, die durch den chirurgischen Plan identifiziert wird;
- von einem Kameraverfolgungssystem eine aktuelle Pose eines chirurgischen Werkzeugs zu erhalten, das verwendet wird, um die Wirbelsäulenimplantatvorrichtung in die Wirbelsäule des Kandidatenpatienten zu implantieren;
- Navigationsinformationen basierend auf einem Vergleich der aktuellen Pose des chirurgischen Werkzeugs (1102) und der geplanten Trajektorie zu erzeugen, wobei die Navigationsinformationen angeben, wie das chirurgische Werkzeug posiert werden muss, um mit der geplanten Trajektorie (1120) ausgerichtet zu werden; und
- mindestens einen ersten Teil der Navigationsinformationen für eine Anzeigevorrichtung bereitzustellen.

6. Chirurgisches System nach Anspruch 5, das ferner betreibbar ist, um: mindestens einen zweiten Teil der Navigationsinformationen für den chirurgischen Roboter (14) bereitzustellen, um die Bewegung eines Roboterarms mit einem Endeffektor zu steuern, der die Bewegung des chirurgischen Werkzeugs führt, wobei der mindestens zweite Teil der Navigationsinformationen angibt, wo der Endeffektor bewegt werden muss, so dass das chirurgische Werkzeug durch den Endeffektor entlang der geplanten Trajektorie für die Implantation der Wirbelsäulenimplantatvorrichtung zu der geplanten Pose der Wirbelsäulenimplantatvorrichtung in die Wirbelsäule des Kandidatenpatienten geführt wird.

7. Chirurgisches System nach Anspruch 1, wobei die Rückmeldungsdaten, die verwendet werden, um das Maschinenlernmodell (1300) zu trainieren, und die das chirurgische Ergebnis des früheren Patienten charakterisieren, postoperative Rückmeldungsdaten umfassen, die eine vom Patienten gemeldete Ergebnismessung oder eine Korrekturmessung der Wirbelsäulenverformung charakterisieren.

8. Chirurgisches System nach Anspruch 1, wobei die Rückmeldungsdaten, die verwendet werden, um das Maschinenlernmodell (1300) zu trainieren, einen Typ des Wirbelsäulenchirurgieverfahrens, das an dem früheren Patienten durchgeführt wird, und eine Typ- und Dimensionsgröße einer Wirbelsäulenimplantatvorrichtung charakterisieren, die in den früheren Patienten implantiert wurde.

9. Chirurgisches System nach Anspruch 8, wobei die Rückmeldungsdaten, die verwendet werden, um das Maschinenlernmodell (1300) zu trainieren, ferner ein Volumen eines Knochentransplantats charakterisieren, das mit der Wirbelsäulenimplantatvorrichtung verwendet wird, wenn sie in den früheren Patienten implantiert wird, und wobei vorzugsweise die Rückmeldungsdaten, die verwendet werden, um das Maschinenlernmodell zu trainieren, ferner mindestens eines von Folgendem charakterisieren:
- Abweichung zwischen einem geplanten Niveau der Wirbelsäulenkorrektur für den früheren Patienten, das während einer präoperativen Phase geplant wurde, und einem erreichten Niveau der Wirbelsäulenkorrektur für den früheren Patienten, das während einer intraoperativen Phase oder postoperativen Phase gemessen wurde;
- Abweichung zwischen einem geplanten chirurgischen Verfahren, das während einer präoperativen Phase geplant wurde, und einem verwendeten chirurgischen Verfahren, das an dem früheren Patienten während einer intraoperativen Phase durchgeführt wurde;
- Abweichung zwischen einer Typ- und Dimensionsgröße einer Wirbelsäulenimplantatvorrichtung, die während einer präoperativen Phase geplant wurde, und einer verwendeten Typ- und Dimensionsgröße einer Wirbelsäulenimplantatvorrichtung, die in den früheren Patienten während einer intraoperativen Phase implantiert wurde;
- Abweichung zwischen einer geplanten Pose einer Wirbelsäulenimplantatvorrichtung zur Fixierung in der Wirbelsäule des früheren Patienten, die während einer präoperativen Phase geplant wurde, und einer verwendeten Pose der Wirbelsäulenimplantatvorrichtung nach der Fixierung in der Wirbelsäule des früheren Patienten während einer intraoperativen Phase; und
- Abweichung zwischen einer geplanten Einführungstrajektorie (1120) für die Implantation der Wirbelsäulenimplantatvorrichtung in die Wirbelsäule des früheren Patienten, die während einer präoperativen Phase geplant wurde, und einer verwendeten Trajektorie, entlang der die Wirbelsäulenimplantatvorrichtung bewegt wurde, wenn sie in die Wirbelsäule des früheren Patienten während einer intraoperativen Phase implantiert wurde.

10. Chirurgisches System nach Anspruch 1, wobei das Maschinenlernmodell umfasst:
- eine neuronale Netzwerkkomponente, umfassend eine Eingangsschicht mit Eingangsknoten, eine Sequenz von verborgenen Schichten, die jeweils eine Vielzahl von Kombinationsknoten aufweisen, und eine Ausgangsschicht mit Ausgangsknoten; und
- mindestens eine Verarbeitungsschaltung, die betreibbar ist, um unterschiedliche Einträge der präoperativen Daten an unterschiedliche der Eingangsknoten des neuronalen Netzwerkmodells bereitzustellen, und um den chirurgischen Plan basierend auf der Ausgabe von Ausgangsknoten der neuronalen Netzwerkkomponente zu erzeugen, und vorzugsweise ferner umfassend eine Rückmeldungstrainingskomponente, die betreibbar ist, um: Gewichte und/oder Auslöseschwellen, die von den Kombinationsknoten der neuronalen Netzwerkkomponente verwendet werden, basierend auf Werten der Rückmeldungsdaten anzupassen.

11. Chirurgisches System nach einem der vorhergehenden Ansprüche, wobei die erweiterte Realität eine Vielzahl von Kameras (1040), ein Mikrofon (1042), einen Gestensensor (1044), einen Posensensor (1046), ein Anzeigemodul (1048), das die Anzeigevorrichtung (1050) enthält, und eine drahtlose oder drahtgebundene Kommunikationsschnittstelle (1052) umfasst, wobei die Kameras (1040) konfiguriert sind, um als der Gestensensor (1044) zu arbeiten, indem sie zur Identifikation Benutzerhandgesten erfassen, die innerhalb des Sichtfelds der Kamera(s) (1040) durchgeführt werden.

## Revendications

1. Système chirurgical comprenant
- un système de guidage chirurgical (1220) pour la navigation assistée par ordinateur pendant la chirurgie de la colonne vertébrale, le système de guidage chirurgical fonctionnant pour :
- obtenir des données de retour d'information d'un ou de plusieurs ordinateurs distribués en réseau (1230) associés à chacun d'une pluralité de patients antérieurs ayant subi une chirurgie de la colonne vertébrale, les données de retour d'information caractérisant les structures géométriques de la colonne vertébrale du patient antérieur, caractérisant une procédure chirurgicale réalisée sur le patient antérieur (1200), caractérisant un dispositif d'implant qui a été implanté chirurgicalement dans la colonne vertébrale du patient antérieur, et caractérisant le résultat chirurgical du patient antérieur (1204) ;
- entraîner un modèle d'apprentissage automatique (1300) sur la base des données de retour d'information ;
- obtenir des données préopératoires (1200) de l'un des ordinateurs du réseau distribué caractérisant les structures géométriques de la colonne vertébrale d'un patient candidat à une intervention chirurgicale planifiée ;
- générer un plan chirurgical pour le patient candidat sur la base du traitement des données préopératoires par le modèle d'apprentissage automatique (1300) ; et
- fournir au moins une portion du plan chirurgical à un dispositif d'affichage pour examen visuel par un utilisateur, dans lequel le plan chirurgical identifie le type et la taille d'un dispositif d'implant rachidien pour l'implantation chirurgicale dans la colonne vertébrale du patient candidat et identifie une trajectoire planifiée (1120) pour l'implantation du dispositif d'implant rachidien ;
- un système de suivi (6 ; 6') permettant de déterminer la pose actuelle d'un outil chirurgical (1102) utilisé pour implanter le dispositif d'implant vertébral dans la colonne vertébrale du patient candidat ; et
- au moins un contrôleur (828, 846) capable de générer des informations de navigation sur la base d'une comparaison entre la pose actuelle de l'outil chirurgical et la trajectoire prévue, les informations de navigation indiquant comment l'outil chirurgical doit être posé pour être aligné sur la trajectoire prévue, et de fournir les informations de navigation à un dispositif d'affichage (34),
le système chirurgical comprend en outre :
- un robot chirurgical (14) incluant
- une base de robot (10),
- un bras robotique connecté à la base du robot, le bras robotique étant configuré pour se connecter à un effecteur (100) qui guide le déplacement de l'outil chirurgical (1102), et
- au moins un moteur connecté de manière opérationnelle pour déplacer le bras du robot (18,20) par rapport à la base du robot,
- dans lequel l'au moins un contrôleur est connecté à l'au moins un moteur et opérationnel pour
- déterminer une pose de l'effecteur par rapport à une pose prévue de l'effecteur tout en guidant le déplacement de l'outil chirurgical le long de la trajectoire prévue pendant l'implantation du dispositif d'implant vertébral, et
- générer des informations de navigation sur la base de la comparaison entre la pose planifiée et la pose déterminée de l'effecteur, les informations de navigation indiquant où l'effecteur doit être déplacé pour s'aligner sur la pose planifiée afin que l'outil chirurgical soit guidé par l'effecteur le long de la trajectoire planifiée vers le patient,
- un casque de réalité étendue (920) incluant le dispositif d'affichage, l'au moins un contrôleur étant opérationnel pour générer une représentation graphique des informations de navigation fournies au dispositif d'affichage du casque XR afin de guider le déplacement de l'outil chirurgical le long de la trajectoire planifiée,
- des réseaux de référence dynamiques (52), DRA, qui sont attachés ou formés sur le casque XR, l'effecteur, le robot chirurgical, l'outil chirurgical et une structure anatomique d'un patient,
- un système de localisation 3D et une plateforme informatique (910) configurée pour recevoir un balayage d'image 3D ou 2D, la plateforme informatique en combinaison avec le système de localisation 3D étant configurée pour suivre en temps réel la pose du DRA de manière à déterminer la pose du DRA dans tout espace multidimensionnel par rapport à la structure anatomique du patient.

2. Système chirurgical de la revendication 1, dans lequel le modèle d'apprentissage automatique est opérationnel pour : traiter les données préopératoires afin de produire le plan chirurgical identifiant le type et la taille d'un dispositif d'implant rachidien proposé pour l'implantation chirurgicale dans la colonne vertébrale du patient candidat.

3. Système chirurgical de la revendication 2, dans lequel le modèle d'apprentissage automatique (1300) est en outre opérationnel pour traiter les données préopératoires afin de produire le plan chirurgical avec une identification supplémentaire d'un niveau estimé de réussite du résultat chirurgical prédit pour le patient candidat à partir de l'implantation chirurgicale du dispositif d'implant vertébral dans la colonne vertébrale du patient candidat, dans lequel le niveau estimé de réussite du résultat chirurgical indique une mesure de résultat rapportée par le patient la plus probable ou une mesure de correction de déformation vertébrale qui sera obtenue par l'implantation chirurgicale du dispositif d'implant vertébral dans la colonne vertébrale du patient candidat.

4. Système chirurgical de la revendication 2, dans lequel le modèle d'apprentissage automatique (1300) est en outre opérationnel pour traiter les données préopératoires afin de produire le plan chirurgical avec une identification supplémentaire d'une pose planifiée pour l'implantation du dispositif d'implant rachidien dans la colonne vertébrale du patient candidat.

5. Système chirurgical de la revendication 4, fonctionnant en outre pour :
- déterminer une trajectoire planifiée (1120) pour l'implantation du dispositif d'implant vertébral à la pose planifiée dans la colonne vertébrale du patient candidat identifié par le plan chirurgical ;
- obtenir, à partir d'un système de suivi par caméra, la pose actuelle d'un outil chirurgical utilisé pour implanter le dispositif d'implant vertébral dans la colonne vertébrale du patient candidat ;
- générer des informations de navigation basées sur la comparaison de la pose actuelle de l'outil chirurgical (1102) et de la trajectoire planifiée, les informations de navigation indiquant comment l'outil chirurgical doit être posé pour être aligné sur la trajectoire planifiée (1120) ; et
- fournir au moins une première partie des informations de navigation à un dispositif d'affichage.

6. Système chirurgical de la revendication 5, fonctionnant en outre pour : fournir au moins une deuxième partie des informations de navigation au robot chirurgical (14) pour contrôler le déplacement d'un bras robotique ayant un effecteur qui guide le déplacement de l'outil chirurgical, dans lequel au moins la deuxième partie des informations de navigation indique où l'effecteur doit être déplacé pour que l'outil chirurgical soit guidé par l'effecteur le long de la trajectoire planifiée pour l'implantation du dispositif d'implant vertébral à la pose planifiée du dispositif d'implant vertébral dans la colonne vertébrale du patient candidat.

7. Système chirurgical de la revendication 1, dans lequel les données de retour d'information utilisées pour entraîner le modèle d'apprentissage automatique (1300) et qui caractérisent le résultat chirurgical du patient précédent, incluent des données de retour d'information postopératoire caractérisant une mesure de résultat rapportée par le patient ou une mesure de correction de la déformation de la colonne vertébrale.

8. Système chirurgical de la revendication 1, dans lequel les données de retour d'information utilisées pour entraîner le modèle d'apprentissage automatique (1300) caractérisent un type de procédure de chirurgie rachidienne réalisée sur le patient précédent et un type et une taille de dimension d'un dispositif d'implant rachidien qui a été implanté sur le patient précédent.

9. Système chirurgical de la revendication 8,
dans lequel les données de retour d'information utilisées pour entraîner le modèle d'apprentissage automatique (1300) caractérisent en outre un volume de greffe osseuse utilisé avec le dispositif d'implant vertébral lorsqu'il a été implanté chez le patient précédent, et dans lequel, de préférence, les données de retour d'information utilisées pour entraîner le modèle d'apprentissage automatique caractérisent en outre au moins l'un des éléments suivants :
- l'écart entre le niveau de correction de la colonne vertébrale prévu pour le patient précédent au cours d'une phase préopératoire et le niveau de correction de la colonne vertébrale atteint pour le patient précédent, mesuré au cours d'une phase peropératoire ou postopératoire ;
- écart entre une procédure chirurgicale planifiée lors d'une phase préopératoire et une procédure chirurgicale utilisée qui a été réalisée sur le patient précédent lors d'une phase peropératoire ;
- écart entre le type et les dimensions d'un dispositif d'implant vertébral planifié au cours d'une phase préopératoire et le type et les dimensions d'un dispositif d'implant vertébral utilisé et implanté chez le patient précédent au cours d'une phase peropératoire ; écart entre la pose planifiée d'un dispositif d'implant vertébral pour la fixation dans la colonne vertébrale du patient précédent planifié au cours d'une phase préopératoire et la pose utilisée du dispositif d'implant vertébral après fixation dans la colonne vertébrale du patient précédent au cours d'une phase peropératoire ; et
- écart entre une trajectoire d'insertion planifiée (1120) pour l'implantation du dispositif d'implant vertébral dans la colonne vertébrale du patient précédent, planifiée au cours d'une phase préopératoire, et une trajectoire utilisée sur laquelle le dispositif d'implant vertébral a été déplacé lorsqu'il a été implanté dans la colonne vertébrale du patient précédent au cours d'une phase peropératoire.

10. Système chirurgical de la revendication 1, dans lequel le modèle d'apprentissage automatique comprend :
- un composant de réseau neuronal incluant une couche d'entrée avec des nœuds d'entrée, une séquence de couches cachées ayant chacune une pluralité de nœuds de combinaison, et une couche de sortie avec des nœuds de sortie ; et
- au moins un circuit de traitement permettant de fournir différentes entrées des données préopératoires à différents nœuds d'entrée du modèle de réseau neuronal et de générer le plan chirurgical sur la base des sorties des nœuds de sortie du composant de réseau neuronal, et comprenant de préférence un composant d'entraînement par retour d'information permettant d'adapter les poids et/ou les seuils de déclenchement utilisés par les nœuds de combinaison du composant de réseau neuronal sur la base des valeurs des données de retour d'information.

11. Système chirurgical selon l'une des revendications précédentes, dans lequel la réalité étendue inclut plusieurs caméras (1040), un microphone (1042), un capteur de gestes (1044), un capteur de pose (1046), un module d'affichage (1048) contenant le dispositif d'affichage (1050), et une interface de communication sans fil ou câblée (1052), les caméras (1040) étant configurées pour fonctionner comme capteur de gestes (1044) en capturant pour identification les gestes de la main de l'utilisateur réalisés dans le champ de vision de la ou des caméra(s) (1040).
